(19) Europäisches Patentamt
European Patent Office
Office européen des brevets

(11) EP 4 424 298 A2

(12) EUROPEAN PATENT APPLICATION

(43) Date of publication:
**04.09.2024 Bulletin 2024/36**

(21) Application number: **24176810.0**

(22) Date of filing: **18.05.2024**

(51) International Patent Classification (IPC):
***A61K 8/27*** *(2006.01)* ***A61K 8/33*** *(2006.01)*
***A61K 8/36*** *(2006.01)* ***A61K 8/37*** *(2006.01)*
***A61K 8/49*** *(2006.01)* ***A61Q 15/00*** *(2006.01)*

(52) Cooperative Patent Classification (CPC):
**A61Q 15/00; A61K 8/27; A61K 8/33; A61K 8/361; A61K 8/37; A61K 8/4973**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **CLARIANT INTERNATIONAL LTD**
**4132 Muttenz (CH)**

(72) Inventors:
- **FRICKE, Tom**
**65926 Frankfurt am Main (DE)**
- **BACK, Ute**
**65926 Frankfurt am Main (DE)**
- **GROHMANN, Jörg**
**65527 Niedernhausen (DE)**
- **RIEDERLE, Petra**
**87637 Seeg (DE)**

(74) Representative: **Clariant Produkte (Deutschland) GmbH**
**Patent Management**
**Industriepark Höchst, G 860**
**65926 Frankfurt am Main (DE)**

# (54) ANTIMICROBIAL COMPOSITIONS

(57) The present invention relates to an antimicrobial composition comprising
(A) at least one compound selected from glyceryl ethers, glyceryl esters, sorbitan esters, isosorbide esters, and N-methyl cyclic glucamides according to formula (I),

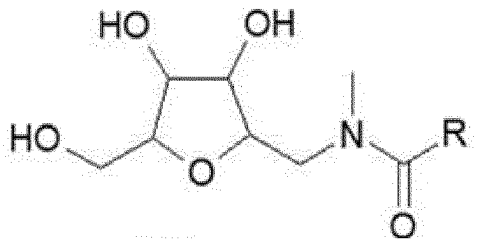

(I),

wherein R is a saturated hydrocarbon chain having 5 to 23 carbon atoms or an unsaturated hydrocarbon chain having 5 to 23 carbon atoms; and
(B) at least one further substance that is active against body odor.

EP 4 424 298 A2

## Description

### FIELD

Embodiments of the present disclosure generally relate to antimicrobial compositions, as well as related methods, formulations, and uses.

### BACKGROUND

Numerous active substances are known that can be used in antiperspirants and deodorants for combating body odor. A disadvantage of using many of these active substances, however, is that their preparation is often complex and based on synthetic raw materials. For some of these active substances, such as *e.g.,* for aluminum-containing substances, alternatives are also sought for toxicological reasons. Additionally, the action of the active against body odor is often in need of improvement, as high use concentrations are necessary for an adequate action. Antiperspirants and deodorants with a long-lasting action in excess of 24 hours are increasingly in demand from consumers.

### SUMMARY

The present disclosure relates to a composition that improves the action of antiperspirants and deodorants in preventing and/or reducing body odor. Embodiments disclosed herein are based on renewable raw materials and are toxicologically acceptable.

In particular, the disclosure is drawn to compositions comprising certain antimicrobial compounds and/or one or more further substances that are active against body odor. It has been found that the antimicrobial activity of such compositions is superior to that of the individual components.

Disclosed herein is an antimicrobial composition comprising

(A) at least one compound selected from glyceryl ethers, glyceryl esters, sorbitan esters, isosorbide esters, and N-methyl cyclic glucamides according to formula (I),

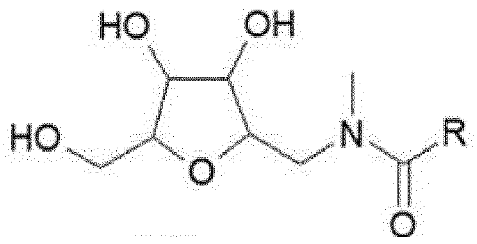
(I),

wherein R is a saturated hydrocarbon chain having 5 to 23 carbon atoms or an unsaturated hydrocarbon chain having 5 to 23 carbon atoms; and

(B) at least one further substance that is active against body odor.

Further described herein is a method of improving the action of an antiperspirant or a deodorant, wherein the method comprises adding to the antiperspirant or the deodorant at least one compound selected from glyceryl ethers, glyceryl esters, sorbitan esters, isosorbide esters, and N-methyl cyclic glucamides according to formula (I),

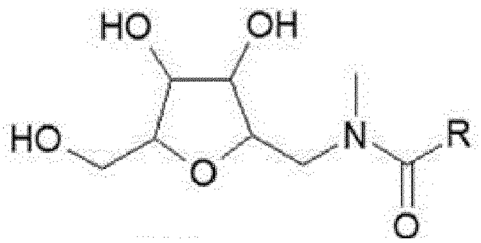
(I)

wherein R is a saturated hydrocarbon chain having 5 to 23 carbon atoms or an unsaturated hydrocarbon chain having 5 to 23 carbon atoms.

Further described herein is the use of at least one compound selected from glyceryl ethers, glyceryl esters, sorbitan esters, isosorbide esters, and N-methyl cyclic glucamides according to formula (I),

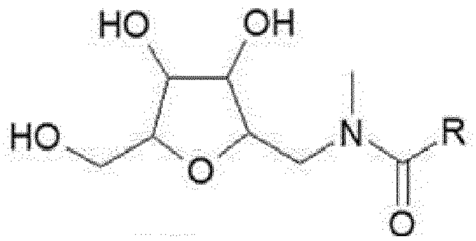

(I),

wherein R is a saturated hydrocarbon chain having 5 to 23 carbon atoms or an unsaturated hydrocarbon chain having 5 to 23 carbon atoms,
to improve the action of an antiperspirant or a deodorant in preventing and/or reducing body odor.

Additional features and advantages will be set forth in the detailed description which follows, and in part will be readily apparent to those skilled in the art from that description or recognized by practicing the embodiments described herein, including the detailed description which follows and the claims. It is to be understood that both the foregoing general description and the following detailed description describe various embodiments and are intended to provide an overview or framework for understanding the nature and character of the claimed subject matter.

## DETAILED DESCRIPTION

Reference will now be made in detail to various embodiments of antimicrobial compositions that improve the action of an antiperspirant or deodorant in preventing and/or reducing body odor.

In this document, including in all embodiments of all aspects of the present disclosure, the following definitions apply unless specifically stated otherwise:
As used herein, the singular forms "a," "an" and "the" include plural. Thus, for example, reference to "a" component includes aspects havingtwo or more such components, unless the context clearly indicates otherwise.

All percentages are by weight (w/w) of the total composition. All ratios are weight ratios. "wt.-%" means percentage by weight. References to 'parts,' e.g., a mixture of 1 part X and 3 parts Y, is a ratio by weight.

"QS" or "QSP" means sufficient quantity for 100 % or for 100 g. +/- indicates the standard deviation. All ranges are inclusive and combinable.

The number of significant digits conveys neither a limitation on the indicated amounts nor on the accuracy of the measurements.

All measurements are understood to be made at 23°C and at ambient conditions, where "ambient conditions" means at 1 atmosphere (atm) of pressure and at 50 % relative humidity.

Herein "min" means "minute" or "minutes."

Herein "h" means "hour" or "hours."

Herein "mol" means "mole."

Herein "g" following a number means "gram" or "grams."

Herein, "comprising" means that other steps and other ingredients can be in addition. "Comprising" encompasses the terms "consisting of" and "consisting essentially of". The compositions, formulations, methods, uses, and processes of the present invention can comprise, consist of, and consist essentially of the elements and limitations of the invention described herein, as well as any of the additional or optional ingredients, components, steps, or limitations described herein. Embodiments and aspects described herein may comprise or be combinable with elements, features or components of other embodiments and/or aspects despite not being expressly exemplified in combination, unless an incompatibility is stated.

Where amount ranges are given, these are to be understood as being the total amount of said ingredient in the composition, or where more than one species fall within the scope of the ingredient definition, the total amount of all ingredients fitting that definition, in the composition.

"Derivatives" includes but is not limited to, amide, ether, ester, amino, carboxyl, acetyl, acid, salt and/or alcohol derivatives of a given compound. In at least one embodiment, "derivatives thereof" means the amide, ether, ester, amino, carboxyl, acetyl, acid, salt and alcohol derivatives.

"Molecular weight" or "M.Wt." or "MW" and grammatical equivalents mean the number average molecular weight.

"Substantially free from" or "substantially free of" means less than 1 %, or less than 0.8 %, or less than 0.5 %, or less than 0.3 %, or about 0 %, by total weight of the composition or formulation.

"Viscosity" is measured at 25°C using a HAAKE Rotation Viscometer VT 550 with cooling/heating vessel and

sensor systems according to DIN 53019 at a shear rate of 12.9 s-1.

"Water-soluble" refers to any material that is sufficiently soluble in water to form a clear solution to the naked eye at a concentration of 0.1 % by weight of the material in water at 25°C. The term "water-insoluble" refers to any material that is not "water-soluble".

As used herein, the "hydroxyl value" of a substance is to be understood as meaning the amount of KOH in milligrams equivalent to the amount of acetic acid bound during the acetylation of 1 g of substance. Suitable determination methods for determining the hydroxyl value are, for instance, DGF C-V 17 a (53), Ph. Eur. 2.5.3 Method A and DIN 53240.

In the present disclosure, hydroxyl values are determined analogously to DIN 53240-2. Here, the following procedure is adopted: 1 g, accurate to 0.1 mg, of the homogenized sample to be measured is weighed out, then 20.00 mL of acetylation mixture (acetylation mixture: 50 mL of acetic anhydride are stirred into 1 L of pyridine) are added. The sample is dissolved completely in the acetylation mixture, if required with stirring and heating. 5 mL of catalyst solution (catalyst solution: 2 g of 4-dimethylaminopyridine are dissolved in 100 mL of pyridine) are added.

The reaction vessel is closed and placed into the water bath, preheated to 55° C, for 10 minutes, with mixing. 10 mL of fully deionized water are then added to the reaction solution, the reaction vessel is closed again, and the mixture is once more allowed to react in the water bath with shaking for 10 minutes. The sample is then cooled to room temperature (25° C). 50 mL of 2-propanol and 2 drops of phenolphthalein are then added. This solution is titrated with aqueous sodium hydroxide solution (aqueous sodium hydroxide solution c = 0.5 mol/L) (Va). Under identical conditions, but without any sample added, the efficacy of the acetylation mixture is determined (Vb).

From the aqueous sodium hydroxide solution consumed in the determination of the efficacy and the titration of the sample, the hydroxyl value (OHV) is calculated using the following formula:

$$OHV = \frac{(Vb - Va) \cdot c \cdot t \cdot M}{E}$$

OHV = hydroxyl value in mg KOH/g substance
Va = aqueous sodium hydroxide solution consumed in mL during the titration of the sample
Vb = aqueous sodium hydroxide solution consumed in mL during the titration of efficacy
c = molar concentration of the aqueous sodium hydroxide solution in mol/L
t = titer of the aqueous sodium hydroxide solution
M = molar mass of KOH = 56.11 g/mol
E = sample weighed out in g
(Vb-Va) is the amount of aqueous sodium hydroxide solution used in mL, which is equivalent to the amount of acetic acid bound during the above-described acetylation of the sample to be measured.

Unless otherwise expressly stated, it is in no way intended that any method set forth herein be construed as requiring that its steps be performed in a specific order, nor that with any apparatus specific orientations be required. Accordingly, where a method claim does not actually recite an order to be followed by its steps, or that any apparatus claim does not actually recite an order or orientation to individual components, or it is not otherwise specifically stated in the claims or description that the steps are to be limited to a specific order, or that a specific order or orientation to components of an apparatus is not recited, it is in no way intended that an order or orientation be inferred, in any respect. This holds for any possible non-express basis for interpretation, including matters of logic with respect to arrangement of steps, operational flow, order of components, or orientation of components; plain meaning derived from grammatical organization or punctuation; and the number or type of embodiments described in the specification.

Described herein are embodiments of an antimicrobial composition. Also described herein are embodiments of a formulation comprising an antimicrobial compound. As used herein, "antimicrobial" means to inhibit the growth of certain microbes, such as odor-causing bacteria or fungus, that are found on the surface of the skin. As used herein, "antimicrobial activity" is the inhibition of the growth of certain microbes, such as odor-causing bacteria or fungus, that are found on the surface of the skin. As used herein, an "antimicrobial agent" is a substance that inhibits the growth of certain microbes, such as odor-causing bacteria or fungus, that are found on the surface of the skin.

Further described herein are embodiments of various uses and methods, such as for improving the effectiveness of an antiperspirant or a deodorant in preventing and/or reducing body odor. As used herein, "improve the action of an antiperspirant or a deodorant" means to enhance the effectiveness of the deodorant or the antiperspirant in preventing and/or reducing body odor.

The antimicrobial composition described herein comprises (A) at least one compound selected from glyceryl ethers, glyceryl esters, sorbitan esters, isosorbide esters, and N-methyl cyclic glucamides according to Formula (I),

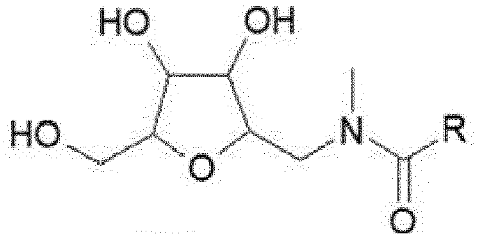

(I),

wherein R is a saturated hydrocarbon chain having 5 to 23 carbon atoms or an unsaturated hydrocarbon chain having 5 to 23 carbon atoms.

In embodiments, the antimicrobial composition described herein comprises (A) at least one glyceryl ether, at least one glyceryl ester, at least one sorbitan ester, at least one isosorbide ester, at least one N-methyl cyclic glucamide according to Formula (I), or any combination of these.

In embodiments, the antimicrobial composition comprises (A) at least one glyceryl ether. In some embodiments, the glyceryl ether is a mono- or diether of glycerin and one or more $C_6$-$C_{20}$ fatty alcohols. In some embodiments, the glyceryl ether is a mono- or diether of glycerin and one or more $C_8$-$C_{14}$ fatty alcohols. In some embodiments, the glyceryl ether is a monoether of glycerin and one or more $C_8$ fatty alcohols. In embodiments, the antimicrobial composition described herein comprises ethylhexylglycerin, methylheptylglycerin, caprylyl glyceryl ether, or mixtures thereof. In embodiments, the antimicrobial composition described herein comprises caprylyl glyceryl ether.

In embodiments, the antimicrobial composition described herein comprises (A) at least one glyceryl ester. In embodiments, the glyceryl ester is glyceryl caprylate or glyceryl caprate. In embodiments, the antimicrobial composition described herein comprises a mixture of glyceryl caprylate and glyceryl caprate.

In embodiments, the antimicrobial composition described herein comprises (A) at least one sorbitan ester. In some embodiments, the sorbitan ester is a mono-, di-, or triester of sorbitan and one or more $C_6$-$C_{20}$ fatty acids. In some embodiments, the sorbitan ester is a mono- or diester of sorbitan and one or more $C_8$-$C_{14}$ fatty acids. In some embodiments, the sorbitan ester is a mono- or diester of sorbitan and caprylic acid. In some embodiments, the antimicrobial composition described herein comprises sorbitan caprylate, sorbitan stearate, sorbitan olivate, sorbitan oleate, sorbitan caprate, sorbitan laurate, sorbitan myristate, sorbitan caproate, or mixtures thereof. In embodiments, the antimicrobial composition described herein comprises sorbitan caprylate. In some embodiments, the antimicrobial composition described herein comprises at least one sorbitan ester with a hydroxyl value of less than or equal to 320, less than or equal to 285, less than or equal to 245, or less than or equal to 225.

In embodiments, the antimicrobial composition described herein comprises (A) at least one isosorbide ester. In some embodiments, the isosorbide ester is a mono- or diester of isosorbide and one or more $C_6$-$C_{20}$ fatty acids. In some embodiments, the isosorbide ester is a mono- or diester of isosorbide and one or more $C_8$-$C_{14}$ fatty acids. In some embodiments, the isosorbide ester is a mono- or diester of isosorbide and caprylic acid. In some embodiments, the antimicrobial composition described herein comprises isosorbide caprylate, isosorbide stearate, isosorbide olivate, isosorbide oleate, isosorbide caprate, isosorbide laurate, isosorbide myristate, isosorbide caproate, or mixtures thereof. In embodiments, the antimicrobial composition described herein comprises isosorbide caprylate. In some embodiments, the antimicrobial composition described herein comprises at least one isosorbide ester with a hydroxyl value of less than or equal to 320, less than or equal to 285, less than or equal to 245, or less than or equal to 225.

In some embodiments, the antimicrobial composition described herein comprises a mixture of at least one sorbitan ester and at least one isosorbide ester. In some embodiments, the mixture of the at least one sorbitan ester and the at least one isosorbide ester has a hydroxyl value that is less than or equal to 320, less than or equal to 285, less than or equal to 245, or less than or equal to 225.

In embodiments, the antimicrobial composition described herein comprises (A) at least one N-methyl cyclic glucamide according to Formula (I),

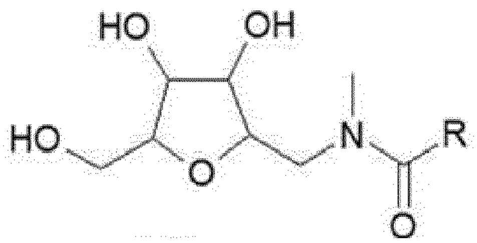

(I),

wherein R is a saturated hydrocarbon chain having 5 to 23 carbon atoms or an unsaturated hydrocarbon chain having

5 to 23 carbon atoms.

In some embodiments, R in Formula (I) is a saturated hydrocarbon chain having 5 to 17 carbon atoms, 8 to 13 carbon atoms, 10 to 12 carbon atoms, or 7 to 9 carbon atoms. In some embodiments, R in Formula (I) is an unsaturated hydrocarbon chain having 5 to 17 carbon atoms, 8 to 13 carbon atoms, 10 to 12 carbon atoms, or 7 to 9 carbon atoms. In some embodiments, R in Formula (I) is $-(CH_2)_6CH_3$, $-(CH_2)_8CH_3$, $-(CH_2)_{10}CH_3$, $-(CH_2)_{12}CH_3$, $-(CH_2)_7CH=CH_2$, or $-(CH_2)_7CH=CHCH_2CH_3$.

In embodiments, the antimicrobial composition described herein comprises a mixture of N-methyl cyclic glucamides according to Formula (I).

In some embodiments, the antimicrobial composition described herein comprises a mixture of at least one N-methyl cyclic glucamide according to Formula (I) wherein R is $-(CH_2)_6CH_3$ and at least one N-methyl cyclic glucamide according to Formula (I) wherein R is $-(CH_2)_8CH_3$. In embodiments, the antimicrobial composition described herein comprises a mixture of N-methyl cyclic glucamides according to Formula (I) wherein R is $-(CH_2)_6CH_3$ and N-methyl cyclic glucamides according to Formula (I) wherein R is $-(CH_2)_8CH_3$ in a weight ratio of from 1:9 to 9:1 or from 3:7 to 7:3.

In some embodiments, the antimicrobial composition described herein comprises a mixture of at least one N-methyl cyclic glucamide according to Formula (I) wherein R is $-(CH_2)_{10}CH_3$ and at least one N-methyl cyclic glucamide according to Formula (I) wherein R is $-(CH_2)_{12}CH_3$. In embodiments, the antimicrobial composition described herein comprises a mixture of N-methyl cyclic glucamides according to Formula (I) wherein R is $-(CH_2)_{10}CH_3$ and N-methyl cyclic glucamides according to Formula (I) wherein R is $-(CH_2)_{12}CH_3$ in a weight ratio of from 1:9 to 9:1 or from 3:7 to 7:3.

In some embodiments, the antimicrobial composition described herein comprises a mixture of at least one N-methyl cyclic glucamide according to Formula (I) wherein R is $-(CH_2)_7CH=CH_2$ and at least one N-methyl cyclic glucamide according to Formula (I) wherein R is $-(CH_2)_7CH=CHCH_2CH_3$. In embodiments, the antimicrobial composition described herein comprises a mixture of N-methyl cyclic glucamides according to Formula (I) wherein R is $-(CH_2)_7CH=CH_2$ and N-methyl cyclic glucamides according to Formula (I) wherein R is $-(CH_2)_7CH=CHCH_2CH_3$ in a weight ratio of from 1:9 to 9:1 or from 3:7 to 7:3.

In some embodiments, the antimicrobial composition described herein comprises a mixture of N-methyl cyclic glucamides according to Formula (I) wherein the R-C=0 residue in Formula (I) is derived from coconut fatty acids. As used herein, the expression "the R-C=0 residue in Formula (I) is derived from coconut fatty acids" means that the carbon chain length distribution of the R-C=0 residues in the mixture of N-methyl cyclic glucamides according to Formula (I) corresponds to the carbon chain length distribution of the fatty acids *(e.g.,* bound as triglycerides) in coconut oil.

In some embodiments, the antimicrobial composition described herein comprises a mixture of N-methyl cyclic glucamides according to Formula (I) wherein the R-C=0 residue in Formula (I) is derived from soybean fatty acids. As used herein, the expression "the R-C=0 residue in Formula (I) is derived from soybean fatty acids" means that the carbon chain length distribution of the R-C=0 residues in the mixture of N-methyl cyclic glucamides according to Formula (I) corresponds to the carbon chain length distribution of the fatty acids *(e.g.,* bound as triglycerides) in soybean oil.

The antimicrobial composition described herein also comprises (B) at least one further substance that is active against body odor. In the context of the present disclosure, a "further substance active against body odor" is understood as meaning a substance that is different from a glyceryl ether, a glyceryl ester, a sorbitan ester, an isosorbide ester, and an N-methyl cyclic glucamide according to Formula (I). A "further substance active against body odor" can include any material that is known or otherwise effective in preventing and/or reducing body odor associated with perspiration.

In embodiments, the antimicrobial composition described herein comprises (B) at least one further substance active against body odor selected from *Acorus gramineus* root/stem/luffa cylindrica fruit/*Camellia sinensis* leaf extract, adipic acid/neopentyl glycol crosspolymer, *Alpinia uralensis* stalk/leaf water, amber powder, ammonium phenolsulfonate, ammonium silver zeolite, ammonium silver zinc aluminium silicate, benzalkonium bromide, benzalkonium cetyl phosphate, benzalkonium chloride, benzalkonium saccharinate, benzethonium chloride, *Boesenbergia pandurata* rhizome extract, bromochlorophene, *Bursera graveolens* fruit oil, butyl acrylate/ethyltrimonium chloride methacrylate/styrene copolymer, t-butyl methylphenoxy phenol, butyloctanoic acid, calcium magnesium silicate, *Callicarpa macrophylia* flower extract, *Candida bombicola*/glucose/methyl rapeseedate ferment, capramidoethyl capramidopropyldimonium methosultate, caprylol gold of pleasure amino acids, caprylyl glycol, *Castanea crenata* (chestnut) pellicle extract, cetylpyridinium chloride, chitosan, chlorophyllin copper complex, chlorothymol, chloroxylenol, *Citrus reticulata* (tangerine) peel oil, cioflucarban, *Coix lacryma-jobi* Ma-Yuen seed/*Pueraria lobata* root/*Gandodenna lucidum* (mushroom) extract, colloidal platinum, *Curcuma heyneana* root powder, cyciopentadecanone, dequalinium chloride, dichlorophene, dichloro-m-xylenol, dimethiconepeg-15 crosspolymer, dimethylbicycloheptyl ethanone, dipotassium capryloyl glutamate, disodium capryloyl glutamate, disoclium dihydroxyethyl sultosuccinylundecylenate, domiphen bromide, ethylhexylglycerin, fermented vegetable, ferric chloride, geranic acid, glyceryl caprylate, glyceryl caprylate/caprate, hexachlorophene, hexanediolpeg-2 cocomonium chloride/tdi copolymer, *Humulus lupulus* (hops) cone extract, hydrolyzed cellulose, hydrolyzed *sasa veitchii* extract, ketoglutaric acid, *Hypericum perforatum* flower/twig extract, laury isoquinolinium bromide, laurylpyridinium chloride, macelignan, *Mentha aquatica* water, methylbenzethonium chloride, 2-methyl 5-cyclohexylpentanol, methyl phenylbutanol, methyl undecylenate, *Michelia champaca* flower oil, *Micrococcus*/*hydrolyzed* nonfat milk ferment octadecen-

edicic acid, octanediol, octenidine HCl, oligopeptide-10, oxidized beta-glucan, *Pandanus amaryilifolius* leaf extract, panduratin A *Pelargonium graveolens* water, phenol, *Phyllostachys edulis* stem extract, *Piper betle* leaf oil, piroctonol, piroctone olamine, polyaminopropyl biguanide stearate, potassium capryloyl glutamate, rapeseed sophorolipids, *Rosmarinus officinalis* (rosemary) flower extract, *Saccharornvoes*/*Persimmon* fruit juice ferment extract, *Saccharomycsi*/*Rhodobacteri*/*Lactobacillus*/*Leuconostoc*/*Streptornyces Griseus*/*Aspergillus*/*Bacillus* ferment filtrate, *Sasa senanensis* leaf extract, *Sasa senanensis* leaf powder, scallop shell powder, shikimic acid, silver citrate, silver chloride (and) titanium dioxide, silver chloride (and) titanium dioxide (and) diethylhexyl sodium sulfosuccinate (and) propylene glycol, silver copper zeolite, silver lactate, sodium bicarbonate, sodium capryloyl glutamate, sodium phenolsulfonate, *Stemmacantha carthamoides* root extract, totarol, triclocarban, triclosan, tricyclodecenyl propionate, triethyl citrate, *Urginea maritima* tuber extract, zeolite, zinc dimethicone PEG-8 succinate, zinc lactate, zinc phenoisulfonate, zinc ricinoleate, zinc silicate, and zirconium powder. In embodiments, the antimicrobial composition described herein comprises zinc ricinoleate, triethyl citrate, or mixtures thereof.

The antimicrobial compositions described herein can be prepared as a blend by methods known in the art, *e.g.,* by mixing the components.

In embodiments, the antimicrobial composition described herein is a blend comprising, based on the total weight of (A) and (B) in the blend, from 10 wt.-% to 90 wt.-%, from 15 wt.-% to 90 wt.-%, from 20 wt.-% to 90 wt.-%, from 25 wt.-% to 90 wt.-%, from 30 wt.-% to 90 wt.-%, from 35 wt.-% to 90 wt.-%, from 40 wt.-% to 90 wt.-%, from 45 wt.-% to 90 wt.-%, from 50 wt.-% to 90 wt.-%, from 55 wt.-% to 90 wt.-%, from 60 wt.-% to 90 wt.-%, from 65 wt.-% to 90 wt.-%, from 70 wt.-% to 90 wt.-%, from 75 wt.-% to 90 wt.-%, from 80 wt.-% to 90 wt.-%, from 85 wt.-% to 90 wt.-%, from 10 wt.-% to 85 wt.-%, from 15 wt.-% to 85 wt.-%, from 20 wt.-% to 85 wt.-%, from 25 wt.-% to 85 wt.-%, from 30 wt.-% to 85 wt.-%, from 35 wt.-% to 85 wt.-%, from 40 wt.-% to 85 wt.-%, from 45 wt.-% to 85 wt.-%, from 50 wt.-% to 85 wt.-%, from 55 wt.-% to 85 wt.-%, from 60 wt.-% to 85 wt.-%, from 65 wt.-% to 85 wt.-%, from 70 wt.-% to 85 wt.-%, from 75 wt.-% to 85 wt.-%, from 80 wt.-% to 85 wt.-%, from 10 wt.-% to 80 wt.-%, from 15 wt.-% to 80 wt.-%, from 20 wt.-% to 80 wt.-%, from 25 wt.-% to 80 wt.-%, from 30 wt.-% to 80 wt.-%, from 35 wt.-% to 80 wt.-%, from 40 wt.-% to 80 wt.-%, from 45 wt.-% to 80 wt.-%, from 50 wt.-% to 80 wt.-%, from 55 wt.-% to 80 wt.-%, from 60 wt.-% to 80 wt.-%, from 65 wt.-% to 80 wt.-%, from 70 wt.-% to 80 wt.-%, from 75 wt.-% to 80 wt.-%, from 10 wt.-% to 75 wt.-%, from 15 wt.-% to 75 wt.-%, from 20 wt.-% to 75 wt.-%, from 25 wt.-% to 75 wt.-%, from 30 wt.-% to 75 wt.-%, from 35 wt.-% to 75 wt.-%, from 40 wt.-% to 75 wt.-%, from 45 wt.-% to 75 wt.-%, from 50 wt.-% to 75 wt.-%, from 55 wt.-% to 75 wt.-%, from 60 wt.-% to 75 wt.-%, from 65 wt.-% to 75 wt.-%, from 70 wt.-% to 75 wt.-%, from 10 wt.-% to 70 wt.-%, from 15 wt.-% to 70 wt.-%, from 20 wt.-% to 70 wt.-%, from 25 wt.-% to 70 wt.-%, from 30 wt.-% to 70 wt.-%, from 35 wt.-% to 70 wt.-%, from 40 wt.-% to 70 wt.-%, from 45 wt.-% to 70 wt.-%, from 50 wt.-% to 70 wt.-%, from 55 wt.-% to 70 wt.-%, from 60 wt.-% to 70 wt.-%, from 65 wt.-% to 70 wt.-%, from 10 wt.-% to 65 wt.-%, from 15 wt.-% to 65 wt.-%, from 20 wt.-% to 65 wt.-%, from 25 wt.-% to 65 wt.-%, from 30 wt.-% to 65 wt.-%, from 35 wt.-% to 65 wt.-%, from 40 wt.-% to 65 wt.-%, from 45 wt.-% to 65 wt.-%, from 50 wt.-% to 65 wt.-%, from 55 wt.-% to 65 wt.-%, from 60 wt.-% to 65 wt.-%, from 10 wt.-% to 60 wt.-%, from 15 wt.-% to 60 wt.-%, from 20 wt.-% to 60 wt.-%, from 25 wt.-% to 60 wt.-%, from 30 wt.-% to 60 wt.-%, from 35 wt.-% to 60 wt.-%, from 40 wt.-% to 60 wt.-%, from 45 wt.-% to 60 wt.-%, from 50 wt.-% to 60 wt.-%, from 55 wt.-% to 60 wt.-%, from 10 wt.-% to 55 wt.-%, from 15 wt.-% to 55 wt.-%, from 20 wt.-% to 55 wt.-%, from 25 wt.-% to 55 wt.-%, from 30 wt.-% to 55 wt.-%, from 35 wt.-% to 55 wt.-%, from 40 wt.-% to 55 wt.-%, from 45 wt.-% to 55 wt.-%, from 50 wt.-% to 55 wt.-%, from 10 wt.-% to 50 wt.-%, from 15 wt.-% to 50 wt.-%, from 20 wt.-% to 50 wt.-%, from 25 wt.-% to 50 wt.-%, from 30 wt.-% to 50 wt.-%, from 35 wt.-% to 50 wt.-%, from 40 wt.-% to 50 wt.-%, from 45 wt.-% to 50 wt.-%, from 10 wt.-% to 45 wt.-%, from 15 wt.-% to 45 wt.-%, from 20 wt.-% to 45 wt.-%,from 25 wt.-% to 45 wt.-%, from 30 wt.-% to 45 wt.-%, from 35 wt.-% to 45 wt.-%, from 40 wt.-% to 45 wt.-%, 10 wt.-% to 40 wt.-%, from 15 wt.-% to 40 wt.-%, from 20 wt.-% to 40 wt.-%, from 25 wt.-% to 40 wt.-%, from 30 wt.-% to 40 wt.-%, from 35 wt.-% to 40 wt.-%, 10 wt.-% to 30 wt.-%, from 15 wt.-% to 30 wt.-%, from 20 wt.-% to 30 wt.-%, from 25 wt.-% to 30 wt.-%, 10 wt.-% to 25 wt.-%, from 15 wt.-% to 25 wt.-%, from 20 wt.-% to 25 wt.-%, 10 wt.-% to 20 wt.-%, or from 15 wt.-% to 20 wt.-%, of (A) the at least one compound selected from glyceryl ethers, glyceryl esters, sorbitan esters, isosorbide esters, and N-methyl cyclic glucamides according to Formula (I).

In embodiments, the antimicrobial composition described herein is a blend comprising, based on the total weight of (A) and (B) in the blend, from 10 wt.-% to 90 wt.-%, from 15 wt.-% to 90 wt.-%, from 20 wt.-% to 90 wt.-%, from 25 wt.-% to 90 wt.-%, from 30 wt.-% to 90 wt.-%, from 35 wt.-% to 90 wt.-%, from 40 wt.-% to 90 wt.-%, from 45 wt.-% to 90 wt.-%, from 50 wt.-% to 90 wt.-%, from 55 wt.-% to 90 wt.-%, from 60 wt.-% to 90 wt.-%, from 65 wt.-% to 90 wt.-%, from 70 wt.-% to 90 wt.-%, from 75 wt.-% to 90 wt.-%, from 80 wt.-% to 90 wt.-%, from 85 wt.-% to 90 wt.-%, from 10 wt.-% to 85 wt.-%, from 15 wt.-% to 85 wt.-%, from 20 wt.-% to 85 wt.-%, from 25 wt.-% to 85 wt.-%, from 30 wt.-% to 85 wt.-%, from 35 wt.-% to 85 wt.-%, from 40 wt.-% to 85 wt.-%, from 45 wt.-% to 85 wt.-%, from 50 wt.-% to 85 wt.-%, from 55 wt.-% to 85 wt.-%, from 60 wt.-% to 85 wt.-%, from 65 wt.-% to 85 wt.-%, from 70 wt.-% to 85 wt.-%, from 75 wt.-% to 85 wt.-%, from 80 wt.-% to 85 wt.-%, from 10 wt.-% to 80 wt.-%, from 15 wt.-% to 80 wt.-%, from 20 wt.-% to 80 wt.-%, from 25 wt.-% to 80 wt.-%, from 30 wt.-% to 80 wt.-%, from 35 wt.-% to 80 wt.-%, from 40 wt.-% to 80 wt.-%, from 45 wt.-% to 80 wt.-%, from 50 wt.-% to 80 wt.-%, from 55 wt.-% to 80 wt.-%, from 60 wt.-% to 80 wt.-%, from 65

wt.-% to 80 wt.-%, from 70 wt.-% to 80 wt.-%, from 75 wt.-% to 80 wt.-%, from 10 wt.-% to 75 wt.-%, from 15 wt.-% to 75 wt.-%, from 20 wt.-% to 75 wt.-%, from 25 wt.-% to 75 wt.-%, from 30 wt.-% to 75 wt.-%, from 35 wt.-% to 75 wt.-%, from 40 wt.-% to 75 wt.-%, from 45 wt.-% to 75 wt.-%, from 50 wt.-% to 75 wt.-%, from 55 wt.-% to 75 wt.-%, from 60 wt.-% to 75 wt.-%, from 65 wt.-% to 75 wt.-%, from 70 wt.-% to 75 wt.-%, from 10 wt.-% to 70 wt.-%, from 15 wt.-% to 70 wt.-%, from 20 wt.-% to 70 wt.-%, from 25 wt.-% to 70 wt.-%, from 30 wt.-% to 70 wt.-%, from 35 wt.-% to 70 wt.-%, from 40 wt.-% to 70 wt.-%, from 45 wt.-% to 70 wt.-%, from 50 wt.-% to 70 wt.-%, from 55 wt.-% to 70 wt.-%, from 60 wt.-% to 70 wt.-%, from 65 wt.-% to 70 wt.-%, from 10 wt.-% to 65 wt.-%, from 15 wt.-% to 65 wt.-%, from 20 wt.-% to 65 wt.-%, from 25 wt.-% to 65 wt.-%, from 30 wt.-% to 65 wt.-%, from 35 wt.-% to 65 wt.-%, from 40 wt.-% to 65 wt.-%, from 45 wt.-% to 65 wt.-%, from 50 wt.-% to 65 wt.-%, from 55 wt.-% to 65 wt.-%, from 60 wt.-% to 65 wt.-%, from 10 wt.-% to 60 wt.-%, from 15 wt.-% to 60 wt.-%, from 20 wt.-% to 60 wt.-%, from 25 wt.-% to 60 wt.-%, from 30 wt.-% to 60 wt.-%, from 35 wt.-% to 60 wt.-%, from 40 wt.-% to 60 wt.-%, from 45 wt.-% to 60 wt.-%, from 50 wt.-% to 60 wt.-%, from 55 wt.-% to 60 wt.-%, from 10 wt.-% to 55 wt.-%, from 15 wt.-% to 55 wt.-%, from 20 wt.-% to 55 wt.-%, from 25 wt.-% to 55 wt.-%, from 30 wt.-% to 55 wt.-%, from 35 wt.-% to 55 wt.-%, from 40 wt.-% to 55 wt.-%, from 45 wt.-% to 55 wt.-%, from 50 wt.-% to 55 wt.-%, from 10 wt.-% to 50 wt.-%, from 15 wt.-% to 50 wt.-%, from 20 wt.-% to 50 wt.-%, from 25 wt.-% to 50 wt.-%, from 30 wt.-% to 50 wt.-%, from 35 wt.-% to 50 wt.-%, from 40 wt.-% to 50 wt.-%, from 45 wt.-% to 50 wt.-%, from 10 wt.-% to 45 wt.-%, from 15 wt.-% to 45 wt.-%, from 20 wt.-% to 45 wt.-%,from 25 wt.-% to 45 wt.-%, from 30 wt.-% to 45 wt.-%, from 35 wt.-% to 45 wt.-%, from 40 wt.-% to 45 wt.-%, 10 wt.-% to 40 wt.-%, from 15 wt.-% to 40 wt.-%, from 20 wt.-% to 40 wt.-%, from 25 wt.-% to 40 wt.-%, from 30 wt.-% to 40 wt.-%, from 35 wt.-% to 40 wt.-%, 10 wt.-% to 30 wt.-%, from 15 wt.-% to 30 wt.-%, from 20 wt.-% to 30 wt.-%, from 25 wt.-% to 30 wt.-%, 10 wt.-% to 25 wt.-%, from 15 wt.-% to 25 wt.-%, from 20 wt.-% to 25 wt.-%, 10 wt.-% to 20 wt.-%, or from 15 wt.-% to 20 wt.-%, of (B) the further substance active against body odor.

In embodiments, the antimicrobial composition described herein is a blend comprising from 10 to 60 wt.-% of (A) and from 40 to 90 wt.-% of (B), such as from 20 to 50 wt.-% of (A) and from 50 to 80 wt.-% of (B) or from 30 to 40 wt.-% of (A) and from 50 to 70 wt.-% of (B), based on the total weight of (A) and (B) in the blend. In embodiments, the antimicrobial composition described herein is a blend comprising from 20 wt.-% to 70 wt.-% of (A) and from 30 wt.-% to 80 wt.-% of (B), such as from 30 wt.-% to 60 wt.-% of (A) and from 40 wt.-% to 70 wt.-% of (B) or from 40 wt.-% to 50 wt.-% of (A) and from 50 wt.-% to 60 wt.-% of (B), based on the total weight of (A) and (B) in the blend. In embodiments, the antimicrobial composition described herein is a blend comprising 50 wt.-% of (A) and 50 wt.-% of (B), based on the total weight of (A) and (B) in the blend.

In embodiments, the antimicrobial composition described herein is a blend comprising a weight-to-weight ratio of (A):(B) of from 10:1 to 1:10, from 9:1 to 1:9, from 8:1 to 1:8, from 7:1 to 1:7, from 6:1 to 1:6, from 5:1 to 1:5, from 4:1 to 1:4, from 3:1 to 1:3, from 2:1 to 1:2, or from 1.5:1 to 1:1.5. In embodiments, the antimicrobial composition described herein is a blend comprising a weight-to-weight ratio of (A):(B) of 1:1.

The present disclosure also relates to a formulation comprising

(A) at least one compound selected from glyceryl ethers, glyceryl esters, sorbitan esters, isosorbide esters, and N-methyl cyclic glucamides according to Formula (I),

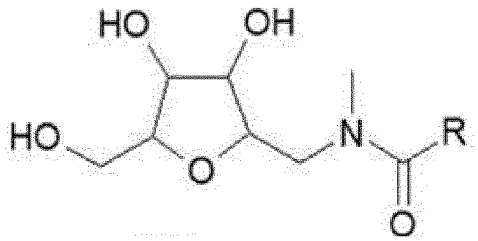 (I),

wherein R is a saturated hydrocarbon chain having 5 to 23 carbon atoms or an unsaturated hydrocarbon chain having 5 to 23 carbon atoms; and

(B) at least one further substance that is active against body odor.

In embodiments of the formulation described herein, (A) and (B) are as defined elsewhere in this disclosure.

In embodiments, the formulation described herein comprises, based on the total weight of the formulation, from 0.1 wt.-% to 10 wt.-%, from 0.2 wt.-% to 10 wt.-%, from 0.5 wt.-% to 10 wt.-%, from 0.8 wt.-% to 10 wt.-%, from 1 wt.-% to 10 wt.-%, from 1.5 wt.-% to 10 wt.-%, from 2 wt.-% to 10 wt.-%, from 2.5 wt.-% to 10 wt.-%, from 3 wt.-% to 10 wt.-%, from 3.5 wt.-% to 10 wt.-%, from 4 wt.-% to 10 wt.-%, from 4.5 wt.-% to 10 wt.-%, from 5 wt.-% to 10 wt.-%, from 5.5 wt.-% to 10 wt.-%, from 6 wt.-% to 10 wt.-%, from 6.5 wt.-% to 10 wt.-%, from 7 wt.-% to 10 wt.-%, from 7.5 wt.-% to 10

wt.-%, 8 wt.-% to 10 wt.-%, from 8.5 wt.-% to 10 wt.-%, from 9 wt.-% to 10 wt.-%, from 9.5 wt.-% to 10 wt.-%, from 0.1 wt.-% to 8 wt.-%, from 0.2 wt.-% to 8 wt.-%, from 0.5 wt.-% to 8 wt.-%, from 0.8 wt.-% to 8 wt.-%, from 1 wt.-% to 8 wt.-%, from 1.5 wt.-% to 8 wt.-%, from 2 wt.-% to 8 wt.-%, from 2.5 wt.-% to 8 wt.-%, from 3 wt.-% to 8 wt.-%, from 3.5 wt.-% to 8 wt.-%, from 4 wt.-% to 8 wt.-%, from 4.5 wt.-% to 8 wt.-%, from 5 wt.-% to 8 wt.-%, from 5.5 wt.-% to 8 wt.-%, from 6 wt.-% to 8 wt.-%, from 6.5 wt.-% to 8 wt.-%, from 7 wt.-% to 8 wt.-%, from 7.5 wt.-% to 8 wt.-%, from 0.1 wt.-% to 6 wt.-%, from 0.2 wt.-% to 6 wt.-%, from 0.5 wt.-% to 6 wt.-%, from 0.8 wt.-% to 6 wt.-%, from 1 wt.-% to 6 wt.-%, from 1.5 wt.-% to 6 wt.-%, from 2 wt.-% to 6 wt.-%, from 2.5 wt.-% to 6 wt.-%, from 3 wt.-% to 6 wt.-%, from 3.5 wt.-% to 6 wt.-%, from 4 wt.-% to 6 wt.-%, from 4.5 wt.-% to 6 wt.-%, from 5 wt.-% to 6 wt.-%, from 5.5 wt.-% to 6 wt.-%, from 0.1 wt.-% to 4 wt.-%, from 0.2 wt.-% to 4 wt.-%, from 0.5 wt.-% to 4 wt.-%, from 0.8 wt.-% to 4 wt.-%, from 1 wt.-% to 4 wt.-%, from 1.5 wt.-% to 4 wt.-%, from 2 wt.-% to 4 wt.-%, from 2.5 wt.-% to 4 wt.-%, from 3 wt.-% to 4 wt.-%, from 3.5 wt.-% to 4 wt.-%, from 0.1 wt.-% to 2 wt.-%, from 0.2 wt.-% to 2 wt.-%, from 0.5 wt.-% to 2 wt.-%, from 0.8 wt.-% to 2 wt.-%, from 1 wt.-% to 2 wt.-%, from 1.5 wt.-% to 2 wt.-%, from 0.1 wt.-% to 1.5 wt.-%, from 0.2 wt.-% to 1.5 wt.-%, from 0.5 wt.-% to 1.5 wt.-%, from 0.8 wt.-% to 1.5 wt.-%, from 1 wt.-% to 1.5 wt.-%, from 0.1 wt.-% to 1 wt.-%, from 0.2 wt.-% to 1 wt.-%, from 0.5 wt.-% to 1 wt.-%, from 0.8 wt.-% to 1 wt.-%, from 0.1 wt.-% to 0.8 wt.-%, from 0.2 wt.-% to 0.8 wt.-%, from 0.5 wt.-% to 0.8 wt.-%, from 0.1 wt.-% to 0.5 wt.-%, from 0.2 wt.-% to 0.5 wt.-%, or from 0.1 wt.-% to 0.2 wt.-%, of (A) the at least one compound selected from glyceryl ethers, glyceryl esters, sorbitan esters, isosorbide esters, and N-methyl cyclic glucamides according to Formula (I).

In embodiments, the formulation described herein comprises, based on the total weight of the formulation, from 0.1 wt.-% to 10 wt.-%, from 0.2 wt.-% to 10 wt.-%, from 0.5 wt.-% to 10 wt.-%, from 0.8 wt.-% to 10 wt.-%, from 1 wt.-% to 10 wt.-%, from 1.5 wt.-% to 10 wt.-%, from 2 wt.-% to 10 wt.-%, from 2.5 wt.-% to 10 wt.-%, from 3 wt.-% to 10 wt.-%, from 3.5 wt.-% to 10 wt.-%, from 4 wt.-% to 10 wt.-%, from 4.5 wt.-% to 10 wt.-%, from 5 wt.-% to 10 wt.-%, from 5.5 wt.-% to 10 wt.-%, from 6 wt.-% to 10 wt.-%, from 6.5 wt.-% to 10 wt.-%, from 7 wt.-% to 10 wt.-%, from 7.5 wt.-% to 10 wt.-%, 8 wt.-% to 10 wt.-%, from 8.5 wt.-% to 10 wt.-%, from 9 wt.-% to 10 wt.-%, from 9.5 wt.-% to 10 wt.-%, from 0.1 wt.-% to 8 wt.-%, from 0.2 wt.-% to 8 wt.-%, from 0.5 wt.-% to 8 wt.-%, from 0.8 wt.-% to 8 wt.-%, from 1 wt.-% to 8 wt.-%, from 1.5 wt.-% to 8 wt.-%, from 2 wt.-% to 8 wt.-%, from 2.5 wt.-% to 8 wt.-%, from 3 wt.-% to 8 wt.-%, from 3.5 wt.-% to 8 wt.-%, from 4 wt.-% to 8 wt.-%, from 4.5 wt.-% to 8 wt.-%, from 5 wt.-% to 8 wt.-%, from 5.5 wt.-% to 8 wt.-%, from 6 wt.-% to 8 wt.-%, from 6.5 wt.-% to 8 wt.-%, from 7 wt.-% to 8 wt.-%, from 7.5 wt.-% to 8 wt.-%, from 0.1 wt.-% to 6 wt.-%, from 0.2 wt.-% to 6 wt.-%, from 0.5 wt.-% to 6 wt.-%, from 0.8 wt.-% to 6 wt.-%, from 1 wt.-% to 6 wt.-%, from 1.5 wt.-% to 6 wt.-%, from 2 wt.-% to 6 wt.-%, from 2.5 wt.-% to 6 wt.-%, from 3 wt.-% to 6 wt.-%, from 3.5 wt.-% to 6 wt.-%, from 4 wt.-% to 6 wt.-%, from 4.5 wt.-% to 6 wt.-%, from 5 wt.-% to 6 wt.-%, from 5.5 wt.-% to 6 wt.-%, from 0.1 wt.-% to 4 wt.-%, from 0.2 wt.-% to 4 wt.-%, from 0.5 wt.-% to 4 wt.-%, from 0.8 wt.-% to 4 wt.-%, from 1 wt.-% to 4 wt.-%, from 1.5 wt.-% to 4 wt.-%, from 2 wt.-% to 4 wt.-%, from 2.5 wt.-% to 4 wt.-%, from 3 wt.-% to 4 wt.-%, from 3.5 wt.-% to 4 wt.-%, from 0.1 wt.-% to 2 wt.-%, from 0.2 wt.-% to 2 wt.-%, from 0.5 wt.-% to 2 wt.-%, from 0.8 wt.-% to 2 wt.-%, from 1 wt.-% to 2 wt.-%, from 1.5 wt.-% to 2 wt.-%, from 0.1 wt.-% to 1.5 wt.-%, from 0.2 wt.-% to 1.5 wt.-%, from 0.5 wt.-% to 1.5 wt.-%, from 0.8 wt.-% to 1.5 wt.-%, from 1 wt.-% to 1.5 wt.-%, from 0.1 wt.-% to 1 wt.-%, from 0.2 wt.-% to 1 wt.-%, from 0.5 wt.-% to 1 wt.-%, from 0.8 wt.-% to 1 wt.-%, from 0.1 wt.-% to 0.8 wt.-%, from 0.2 wt.-% to 0.8 wt.-%, from 0.5 wt.-% to 0.8 wt.-%, from 0.1 wt.-% to 0.5 wt.-%, from 0.2 wt.-% to 0.5 wt.-%, or from 0.1 wt.-% to 0.2 wt.-%, of (B) the further substance active against body odor.

In embodiments, the formulation described herein comprises from 0.1 wt.-% to 10 wt.-% of (A) and from 0.1 wt.-% to 10 wt.-% of (B), based on the total weight of the formulation. In embodiments, the formulation described herein comprises from 0.1 wt.-% to 10 wt.-% of (A) and from 0.2 wt.-% to 8 wt.-% of (B), based on the total weight of the formulation. In embodiments, the formulation described herein comprises from 0.1 wt.-% to 10 wt.-% of (A) and from 0.5 wt.-% to 6 wt.-% of (B), based on the total weight of the formulation. In embodiments, the formulation described herein comprises from 0.1 wt.-% to 10 wt.-% of (A) and from 1 wt.-% to 4 wt.-% of (B), based on the total weight of the formulation.

In embodiments, the formulation described herein comprises from 0.2 wt.-% to 8 wt.-% of (A) and from 0.1 wt.-% to 10 wt.-% of (B), based on the total weight of the formulation. In embodiments, the formulation described herein comprises from 0.2 wt.-% to 8 wt.-% of (A) and from 0.2 wt.-% to 8 wt.-% of (B), based on the total weight of the formulation. In embodiments, the formulation described herein comprises from 0.2 wt.-% to 8 wt.-% of (A) and from 0.5 wt.-% to 6 wt.-% of (B), based on the total weight of the formulation. In embodiments, the formulation described herein comprises from 0.2 wt.-% to 8 wt.-% of (A) and from 1 wt.-% to 4 wt.-% of (B), based on the total weight of the formulation.

In embodiments, the formulation described herein comprises from 0.5 wt.-% to 6 wt.-% of (A) and from 0.1 wt.-% to 10 wt.-% of (B), based on the total weight of the formulation. In embodiments, the formulation described herein comprises from 0.5 wt.-% to 6 wt.-% of (A) and from 0.2 wt.-% to 8 wt.-% of (B), based on the total weight of the formulation. In embodiments, the formulation described herein comprises from 0.5 wt.-% to 6 wt.-% of (A) and from 0.5 wt.-% to 6 wt.-% of (B), based on the total weight of the formulation. In embodiments, the formulation described herein comprises from 0.5 wt.-% to 6 wt.-% of (A) and from 1 wt.-% to 4 wt.-% of (B), based on the total weight of the formulation.

In embodiments, the formulation described herein comprises from 1 wt.-% to 4 wt.-% of (A) and from 0.1 wt.-

% to 10 wt.-% of (B), based on the total weight of the formulation. In embodiments, the formulation described herein comprises from 1 wt.-% to 4 wt.-% of (A) and from 0.2 wt.-% to 8 wt.-% of (B), based on the total weight of the formulation. In embodiments, the formulation described herein comprises from 1 wt.-% to 4 wt.-% of (A) and from 0.5 wt.-% to 6 wt.-% of (A) and from 1 wt.-% to 4 wt.-% of (B), based on the total weight of the formulation. In embodiments, the formulation described herein comprises from 1 wt.-% to 4 wt.-% of (A) and from 1 wt.-% to 4 wt.-% of (B), based on the total weight of the formulation.

Embodiments of the formulation described herein comprise (A) and (B) in a weight-to-weight ratio of from 10:1 to 1:10 of from 9:1 to 1:9, from 8:1 to 1:8, from 7:1 to 1:7, from 6:1 to 1:6, from 5:1 to 1:5, from 4:1 to 1:4, from 3:1 to 1:3, from 2:1 to 1:2 or from 1.5:1 to 1:1.5. In some embodiments, the formulation described herein comprises a weight-to-weight ratio of (A):(B) of 1:1.

In various embodiments, the formulation is a leave-on skin care formulation, such as a deodorant or an antiperspirant. In some embodiments, the deodorant or antiperspirant is in the form of a stick, roll-on, spray, gel, lotion, or cream. In embodiments, the deodorant or antiperspirant is formulated on an aqueous or aqueous-alcoholic basis or is in the form of an emulsion or dispersion. In some embodiments, the deodorant or antiperspirant is in the form of oil-in-water emulsion. The deodorant or antiperspirant has a pH value of from 2 to 11, from 4.5 to 8.5, or from 5.0 to 6.5.

In embodiments, the formulation described herein comprises at least one additive common in cosmetology, pharmacy, and dermatology, which are hereinafter called auxiliaries. In some embodiments, the formulation comprises an auxiliary. In embodiments, the auxiliary is cosmetically acceptable. In some embodiments, the auxiliary is selected from the group consisting of oily substances, waxes, emulsifiers, coemulsifiers, solubilizers, cationic polymers, film formers, superfatting agents, refatting agents, foam stabilizers, stabilizers, active biogenic substances, preservatives, preservation boosting ingredients, anti-fungal substances, anti-dandruff agents, dyes or pigments, particulate substances, opacifiers, abrasives, absorbents, anticaking agents, bulking agents, pearlizing agents, direct dyes, perfumes or fragrances, carriers, solvents or diluents, propellants, functional acids, active ingredients, skin-brightening agents, self-tanning agents, exfoliants, enzymes, anti-acne agents, deodorants and anti-perspirants, viscosity modifiers, thickening and gelling agents, pH adjusting agents, buffering agents, anti-oxidants, chelants, astringents, sunscreens, sun protection agents, UV filters, skin conditioning agents, emollients, humectants, occlusive agents, pediculocides, anti-foaming agents, flavouring agents, electrolytes, oxidizing agents, reducing agents, and combinations thereof.

In embodiments, the formulation described herein comprises one or more oily substance or wax. In certain embodiments, the formulation described herein comprises an oily substance or wax, wherein the oily substance or wax are selected from the group consisting of silicone oils, volatile or nonvolatile, linear, branched or cyclic, optionally with organic modification; phenylsilicones; silicone resins and silicone gums; mineral oils such as paraffin oil or vaseline oil; oils of animal origin such as perhydrosqualene, lanolin; oils of plant origin such as liquid triglycerides, *e.g.,* sunflower oil, corn oil, soybean oil, rice oil, jojoba oil, babassu oil, pumpkin oil, grapeseed oil, sesame oil, walnut oil, apricot oil, macadamia oil, avocado oil, sweet almond oil, lady's-smock oil, castor oil, triglycerides of caprylic/capric acids, olive oil, peanut oil, rapeseed oil, argan oil, abyssinian oil, and coconut oil; synthetic oils such as purcellin oil, isoparaffins, linear and/or branched fatty alcohols and fatty acid esters, preferably guerbet alcohols having 6 to 18, preferably 8 to 10, carbon atoms; esters of linear ($C_6$-$C_{13}$) fatty acids with linear ($C_6$-$C_{20}$) fatty alcohols; esters of branched ($C_6$-$C_{13}$) carboxylic acids with linear ($C_6$-$C_{20}$) fatty alcohols, esters of linear ($C_6$-$C_{18}$) fatty acids with branched alcohols, especially 2-ethylhexanol; esters of linear and/or branched fatty acids with polyhydric alcohols (such as dimerdiol or trimerdiol, for example) and/or guerbet alcohols; triglycerides based on ($C_6$-$C_{10}$) fatty acids; esters such as dioctyl adipate, diisopropyl dimer dilinoleate; propylene glycols/dicaprylate or waxes such as beeswax, paraffin wax or microwaxes, alone or in combination with hydrophilic waxes, such as cetylstearyl alcohol, for example; fluorinated and perfluorinated oils; fluorinated silicone oils; mixtures of the aforementioned compounds.

In embodiments, the formulation described herein comprises one or more emollients. Suitable emollients include, but are not limited to, propylene glycol, polypropylene glycol (like dipropylene glycol, tripropylene glycol, etc.), diethylene glycol, triethylene glycol, PEG-4, PEG-8, 1,2 pentanediol, 1,2 hexanediol, hexylene glycol, glycerin, $C_2$ to $C_{20}$ monohydric alcohols, $C_2$ to $C_{40}$ dihydric or polyhydric alcohols, alkyl ethers of polyhydric and monohydric alcohols, volatile silicone emollients such as cyclopentasiloxane, nonvolatile silicone emollients such as dimethicone, mineral oils, polydecenes, petrolatum, highly branched hydrocarbons, and non-polar fatty acid and fatty alcohol esters, and mixtures thereof and mixtures thereof. In embodiments, the formulation described herein comprises, based on the total weight of the composition, from 0.5 wt.-% to 25 wt.-%, from 1.0 wt.-% to 20 wt%, from 1.5 wt.-% to 15 wt%, or from 2 wt.-% to 10 wt% of at least one emollient.

In embodiments, the formulation described herein comprises one or more emulsifiers. Suitable emulsifiers can include any of a wide variety of nonionic, cationic, anionic, and zwitterionic emulsifiers. Suitable emulsifier types include esters of glycerin, esters of propylene glycol, fatty acid esters of polyethylene glycol, fatty acid esters of polypropylene glycol, esters of sorbitol, esters of sorbitan anhydrides, carboxylic acid copolymers, esters and ethers of glucose, ethoxylated ethers, ethoxylated alcohols, alkyl phosphates, polyoxyethylene fatty ether phosphates, fatty acid amides, acyl lactylates, soaps and mixtures thereof. Suitable emulsifiers can include, but are not limited to, polyethylene glycol 20

sorbitan monolaurate (Polysorbate 20), polyethylene glycol 5 soya sterol, Steareth-20, Ceteareth-20, PPG-2 methyl glucose ether distearate, Ceteth-10, Polysorbate 80, cetyl phosphate, potassium cetyl phosphate, diethanolamine cetyl phosphate, Polysorbate 60, glyceryl stearate, PEG-100 stearate, and mixtures thereof. In embodiments, the formulation described herein comprises, based on the total weight of the composition, from 0.5 wt.-% to 20 wt.-%, from 1.0 wt.-% to 18 wt%, from 1.5 wt.-% to 15 wt%, from 2 wt.-% to 10 wt%, or from 2.5 wt% to 8 wt% of at least one emulsifier.

In embodiments, the formulation described herein comprises one or more humectant or moisturizing materials. Suitable materials include, but are not limited to, urea; guanidine; glycolic acid and glycolate salts (*e.g.,* ammonium and quaternary alkyl ammonium); lactic acid and lactate salts (*e.g.,* ammonium and quaternary alkyl ammonium); aloe vera in any of its variety of forms (*e.g.,* aloe vera gel); polyhydroxy alcohols such as sorbitol, glycerol, hexanetriol, propylene glycol, butylene glycol, hexylene glycol and the like; polyethylene glycols; sugars and starches; sugar and starch derivatives (*e.g.,* alkoxylated glucose); hyaluronic acid; lactamide monoethanolamine; acetamide monoethanolamine: and mixtures thereof. In some embodiments, the formulation described herein comprise glycerol, butylene glycol, hexylene glycol, or mixtures thereof. In embodiments, the formulation described herein comprises, based on the total weight of the composition, from 0.5 wt.-% to 10 wt.-%, from 1.0 wt.-% to 8 wt%, from 1.5 wt.-% to 6 wt%, or from 2 wt.-% to 4 wt% of at least one humectant or moisturizer.

In embodiments, the formulation described herein comprises one or more viscosity modifiers or thickening and/or gelling agents. The viscosity-modifying substance is preferably a thickening polymer. In certain embodiments, the thickening polymer selected from the group consisting of: copolymers of at least one first monomer type, which is chosen from acrylic acid and methacrylic acid, and at least one second monomer type, which is chosen from esters of acrylic acid and ethoxylated fatty alcohol, crosslinked polyacrylic acid, crosslinked copolymers of at least one first monomer type, which is chosen from acrylic acid and methacrylic acid, and at least one second monomer type, which is chosen from esters of acrylic acid with $C_{10}$- to $C_{30}$-alcohols; copolymers of at least one first monomer type, which is chosen from acrylic acid and methacrylic acid, and at least one second monomer type, which is chosen from esters of itaconic acid and ethoxylated fatty alcohol; copolymers of at least one first monomer type, which is chosen from acrylic acid and methacrylic acid, at least one second monomer type, which is chosen from esters of itaconic acid and ethoxylated $C_{10}$-to $C_{30}$-alcohol and a third monomer type, chosen from $C_1$- to $C_4$-aminoalkyl acrylates; copolymers of two or more monomers chosen from acrylic acid, methacrylic acid, acrylic esters and methacrylic esters; copolymers of vinylpyrrolidone and ammonium acryloyldimethyltaurate; copolymers of ammonium acryloyldimethyltaurate and monomers chosen from esters of methacrylic acid and ethoxylated fatty alcohols, hydroxyethylcellulose, hydroxypropylcellulose, hydroxypropylguar, glyceryl polyacrylate, glyceryl polymethacrylate, copolymers of at least one $C_2$-, $C_3$- or $C_4$-alkylene and styrene, polyurethanes, hydroxypropyl starch phosphate, polyacrylamide, copolymer of maleic anhydride and methyl vinyl ether crosslinked with decadiene, carob seed flour, gums such as guar gum, karaya gum, xanthan gum or dehydroxanthan gum, carrageenan, hydrolyzed corn starch; copolymers of polyethylene oxide, fatty alcohols and saturated methylenediphenyl diisocyanate (*e.g.,* PEG-150/stearyl alcohol/SMDI copolymer), and mixtures thereof. In a preferred embodiment, the viscosity modifier or thickening and/or gelling agent is selected from the group consisting of carbomers, acrylates copolymers, xanthan gum, hydroxyethylcellulose, laureth-2, and combinations thereof. In embodiments, the formulation described herein comprises, based on the total weight of the composition, from 0.01 wt.-% to 15 wt.-%, from 0.1 wt.-% to 10 wt.-%, from 0.5 wt.-% to 5 wt.-% of at least one viscosity modifier or thickening and/or gelling agent.

In embodiments, the formulation described herein comprises one or more carriers, solvents, or diluents. In embodiments, the formulation described herein comprises a solvent, wherein the solvent comprises water and/or alcohol. Solvent is useful for providing the compounds used in present invention in liquid form. In certain embodiments, the solvent is cosmetically acceptable. In embodiments, the formulation described herein comprises at least 10 wt.-% water. Water is useful for economic reasons but also because it is cosmetically acceptable. Optionally the compositions comprise water-miscible or water-soluble solvents such as lower alkyl alcohols. In certain embodiments, the formulation as otherwise described herein comprises $C_1$-$C_5$ alkyl monohydric alcohols, preferably $C_2$-$C_3$ alkyl alcohols. The alcohols which may be present are, in particular, lower monohydric or polyhydric alcohols having 1 to 4 carbon atoms customarily used for cosmetic purposes, such as preferably ethanol and isopropanol. Optionally, the composition comprises a water-soluble polyhydric alcohol. In certain embodiments, the water-soluble polyhydric alcohols are polyhydric alcohols having two or more hydroxyl groups in the molecule. In certain embodiments, the water-soluble polyhydric alcohol is selected from the group consisting of: dihydric alcohols such as ethylene glycol, propylene glycol, trimethylene glycol, 1,2-butylene glycol, 1,3-butylene glycol, 1,4-butylene glycol, tetramethylene glycol, 2,3-butylene glycol, pentamethylene glycol, 2-butene-1,4-diol, hexylene glycol, octylene glycol; trihydric alcohols such as glycerine, trimethylol propane, 1,2,6-hexanetriol and the like; tetrahydric alcohols such as penthaerythritol; pentahydric alcohols such as xylytol, etc.; hexahydric alcohols such as sorbitol, mannitol; polyhydric alcohol polymers such as diethylene glycol, dipropylene glycol, polyethylene glycol, polypropylene glycol, tetraethylene glycol, diglycerine, polyethylene glycol, triglycerine, tetraglycerine, polyglycerine; dihydric alcohol alkyl ethers such as ethylene glycol monomethyl ether, ethylene glycol monoethyl ether, ethylene glycol monobutyl ether, ethylene glycol monophenyl ether, ethylene glycol monohexyl ether, ethylene glycol mono-2-methylhexyl ether, ethylene glycol isoamyl ether, ethylene glycol benzyl ether, ethylene glycol isopropyl ether,

ethylene glycol dimethyl ether, ethylene glycol diethyl ether, ethylene glycol dibutyl ether; dihydric alcohol alkyl ethers such as diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methyl ethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, dipropylene glycol butyl ether; dihydric alcohol ether esters such as ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, propylene glycol monophenyl ether acetate; glycerine monoalkyl ethers such as xyl alcohol, selachyl alcohol, batyl alcohol; sugar alcohols such as sorbitol, maltitol, maltotriose, mannitol, sucrose, erythritol, glucose, fructose, starch sugar, maltose, xylytose, starch sugar reduced alcohol, glysolid, tetrahydrofurfuryl alcohol, POE tetrahydrofurfuryl alcohol, POP butyl ether, POP POE butyl ether, tripolyoxypropylene glycerine ether, POP glycerine ether, POP glycerine ether phosphoric acid, POP POE pentanerythritol ether, and mixtures thereof. In a preferred embodiment, the composition comprises a solvent selected from the group consisting of water, glycols, ethanol, and combinations thereof. In a preferred embodiment, the composition comprises an aqueous, alcoholic or aqueous-alcoholic solvent, and wherein the aqueous, alcoholic or aqueous-alcoholic solvent comprises water, ethanol, propanol, isopropanol, 1,2-propylene glycol, 1,3-propylene glycol, isobutanol, butanol, butyl glycol, butyl diglycol, glycerol, or a mixture thereof; preferably wherein the aqueous, alcoholic or aqueous-alcoholic solvent comprises water, ethanol, propanol, isopropanol, 1,2-propylene glycol, 1,3-propylene glycol, glycerol, or mixtures thereof; more preferably wherein the aqueous, alcoholic or aqueous-alcoholic solvent comprises water, isopropanol, 1,2-propylene glycol, 1,3-propylene glycol, or mixtures thereof; even more preferably wherein the aqueous, alcoholic or aqueous-alcoholic solvent consists of water or consists of a mixture of water and an alcohol wherein the alcohol is selected from the group consisting of isopropanol, 1,2-propylene glycol and 1,3-propylene glycol. Natural solvents can also be used. In certain embodiments, the formulation described herein comprises a solvent selected from the group consisting of plant oil, honey, plant-derived sugar compositions, and mixtures thereof. In embodiments, the formulation described herein comprises, based on the total weight of the composition, from 0.5 wt.-% to 90 wt.-%, from 1.0 wt.-% to 80 wt.-%, from 5 wt.-% to 70 wt.-%, or from 10 wt.-% to 60 wt% of at least one carrier, solvent, and/or diluent.

In embodiments, the formulation described herein comprises one or more skin conditioning agents. Skin conditioning agents such as emollients, humectants and occlusive agents are ingredients which help to maintain the soft and smooth appearance of the skin or which help to improve the condition of dry or damaged skin. In embodiments, the skin conditioning agent is selected from the group consisting of oily substances, functional acids or active ingredients, fatty acid N-alkylpolyhydroxyalkyl amides, fatty acids, triglycerides, panthenol, allantoin, bisabolol, glycerol, sorbitol, urea and derivatives thereof, trehalose, erythrulose, pyrrolidone carboxylic acid (PCA) and its salts, polyglucuronic acid, gluconolactone, petrolatum, ubichinon-10 and ubiquinol. In embodiments, the skin conditioning agent is selected from the group consisting of urea, glycerine, pyrrolidone carboxylic acid (PCA) and its salts, panthenol, petrolatum, and combinations thereof. In embodiments, the formulation described herein comprises, based on the total weight of the composition, from 0.001 wt.-% to 5.0 wt.-%, from 0.05 wt.-% to 3.0 wt.-%, or from 0.1 wt.-% to 1.0 wt.-% of at least one skin conditioning agent.

In embodiments, the formulation described herein comprises one or more alkalizing agents or pH adjusting agents. In embodiments, ammonia or caustic soda is suitable, but water-soluble, physiologically tolerable salts of organic and inorganic bases can also be considered. Optionally, the pH adjusting agent is selected from ammonium hydrogen carbonate, ammonia, monoethanolamine, ammonium hydroxide, ammonium carbonate. In certain embodiments, the alkalizing agents is selected from the group consisting of 2-amino-2-methyl-1-propanol, 2-amino-2-methyl-1,3-propanediol, 2-amino-2-ethyl-1,3-propanediol, tris(hydroxyl-methyl)-aminomethane, 2-amino-1-butanole, tris-(2-hydroxypropyl)-amine, 2,2-iminobisethanol, lysine, iminourea (guanidine carbonate), tetrahydro-1,4-oxazine, 2-amino-5-guanidinvaleric acid, 2-aminoethansulfonic acid, diethanolamine, triethanolamine, N-methyl ethanolamine, isopropanolamine, diisopropanolamine, triisopropanolamine, glucamine, sodium hydroxide, potassium hydroxide, lithium hydroxide and magnesium oxide, and mixtures thereof. To establish an acidic pH value, and acid can be included. In embodiments, the formulation described herein comprises an acid selected from the group consisting of hydrochloric acid, phosphoric acid, acetic acid, formic acid, sulfuric acid, hydrochloric acid, citric acid, ascorbic acid, and mixtures thereof. Citric acid is most preferred in that it has high consumer acceptance. In certain embodiments, the acidic pH is adjusted with a buffer such as a phosphate buffer, a TRIS buffer or a citric buffer. The buffers may be used alone or in combination with an acid. In a preferred embodiment, the alkalizing or pH adjusting agent is selected from the group consisting of triethanolamine, sodium hydroxide, lactic acid, citric acid, and combinations thereof. In embodiments, the formulation described herein comprises, based on the total weight of the composition, from 0.001 wt.-% to 5.0 wt.-%, from 0.01 wt.-% to 3.0 wt.-%, or from 0.1 wt.-% to 1.0 wt.-% of at least one alkalizing or pH adjusting agent.

In embodiments, the formulation described herein comprises at least one antioxidant. In some embodiments,

the antioxidant is selected from the group consisting of amino acids, peptides, sugars, imidazoles, carotinoids, carotenes, chlorogenic acid, lipoic acid, thiols, thiol glycosyl esters, thiol N-acetyl esters, thiol methyl esters, thiol ethyl esters, thiol propyl esters, thiol amyl esters, thiol butyl esters, thiol lauryl esters, thiol palmitoyl esters, thiol oleyl esters, thiol linoleyl esters, thiol cholesteryl esters, thiol glyceryl esters, dilaurylthiodipropionate, distearylthiodipropionate, thiodipropionic acid, metal chelators, hydroxy acids, fatty acids, folic acids, vitamin C, tocopherol, vitamin A, stilbenes, derivatives and combinations thereof. In some embodiments, the antioxidant is selected from the group consisting of glycine, histidine, tyrosine, tryptophan, urocaninic acid, D,L-carnosine, D-carnosine, L-carnosine, beta-carotene, alpha-carotene, lycopene, dihydrolipoic acid, aurothioglucose, propylthiouracil, thioredoxine, glutathione, cysteine, cystine, cystamine, buthioninsulfoximine, homocysteinsulfoximine, buthioninsulfone, penta-, hexa-, heptathioninsulfoximine, hydroxyfatty acids, palmitic acid, phytinic acid, lactoferrin, citric acid, lactic acid, malic acid, humic acid, bile acid, bilirubin, biliverdin, EDTA, EGTA, linoleic acid, linolenic acid, oleic acid, butylhydroxyanisol, trihydroxybutyrophenone, ubichinon, ubichinol, ascorbylpalmitate, Mg-ascorbylphosphate, ascorbylacetate, vitamin E acetate, vitamin A palmitate, carnosine, mannose, ZnO, $ZnSO_4$, selenium methionine, stilbenes, superoxide dismutase, and combinations thereof. In certain embodiments, the antioxidant is selected from the group consisting of vitamin A, vitamin A derivatives, vitamin E, vitamin E derivatives, and combinations thereof. In embodiments, the formulation described herein comprises, based on the total weight of the composition, from 0.001 wt % to 10 wt %, from 0.05 wt % to 5.0 wt %, from 0.1 wt % to 3.0 wt %, or from 0.05 wt % to 1.0 wt % of at least one antioxidant.

In embodiments, the formulation described herein comprises at least one preservative. In some embodiments, the preservative can be one or more of benzyl alcohol, Piroctone Olamine, phenoxyethanol, parabens, pentanediol, benzoic acid/sodium benzoate, and sorbic acid/potassium sorbate. Other organic acids can also be used to provide antimicrobial protection. In some embodiments, the preservative can be one or more of phenoxyethanol, benzyl paraben, butyl paraben, ethyl paraben, isobutyl paraben, isopropyl paraben, methyl paraben, propyl paraben, iodopropynyl butylcarbamate, methyldibromoglutaronitrile, DMDM hydantoin and combinations thereof. In some embodiments, the formulation described herein comprises a preservative selected from the group consisting of cetyltrimethyl ammonium chloride, cetylpyridinium chloride, benzethonium chloride, diisobutylethoxyethyldimethyl benzylammonium chloride, sodium N-lauryl sarcosinate, sodium-N-palmethyl sarcosinate, lauroyl sarcosine, N-myristoylglycine, potassium-N-laurylsarcosine, trimethylammonium chloride, sodium aluminium chlorohydroxylactate, triethyl citrate, tricetylmethylammonium chloride, 2,4,4'-trichloro-2'-hydroxydiphenylether (Triclosan), phenoxyethanol, 1,5-pentandiol, 1,6-hexandiol, 3,4,4'-trichlorocarbanilide (Triclocarban), diaminoalkylamide, L-lysine hexadecylamide, heavy metal citrate salts, salicylate, piroctose, zinc salts, pyrithione and its heavy metal salts, zinc pyrithione, zinc phenol sulfate, farnesol, ketoconazol, oxiconazol, bifonazole, butoconazole, cloconazole, clotrimazole, econazole, enilconazole, fenticonazole, isoconazole, miconazole, sulconazole, tioconazole, fluconazole, itraconazole, terconazole, naftifine, terbinafine, selenium disulfide, methylchloroisothiazolinone, methylisothiazolinone, methyldibromo glutaronitrile, silver chloride (AgCl), diazolidinyl urea, imidazolidinyl urea, dehydroacetic acid, undecylenic acid, chlorphenesin, proprionic acid, salicylic acid, chloroxylenol, sodium salts of diethylhexylsulfosuccinate, sodiumbenzoate, phenoxyethanol, (RS)-1-(4-chlorophenoxy)-1-imidazol-1-yl-3,3-dimethylbutan-2-one (climbazole), benzyl alcohol, phenoxyisopropanol, parabens such as butyl-, ethyl-, methyl- and propylparaben and their salts, 2-Bromo-2-nitropropane-1,3-diol, polyaminopropyl biguanide, phenoxyisopropanol, iodopropynyl butylcarbamate, benzalkonium chloride, benzethonium chloride, pentandiol, 1,2-octanediol, ethylhexylglycerin, sorbic acid, benzoic acid, lactic acid, imidazolidinyl urea, diazolidinyl urea, dimethylol dimethyl hydantoin (DMDMH), chlorhexidine, sodium salts of hydroxymethyl glycinate, hydroxyethylglycine of sorbic acid, and combinations thereof. In embodiments, the formulation described herein comprises, based on the total weight of the composition, from 0.01 wt.-% to 5.0 wt % wt.-%, or from 0.05 wt.-% to 1.0 wt.-% of at least one preservative.

In embodiments, the formulation described herein comprises a propellant. In embodiments, the propellant is selected from compressed gas propellants and liquefied gas propellants. In embodiments, the compressed gas propellants are selected from the group consisting of air, nitrogen ($N_2$), nitrous oxide ($N_2O$), carbon dioxide ($CO_2$), and mixtures thereof; preferably air, nitrogen ($N_2$), and mixtures thereof; most preferably nitrogen ($N_2$). In embodiments, the liquefied gas propellants are selected from the group consisting of dimethylether (DME), 1,1-difluoroethane (HFC-152a), 1,1,1,2-tetrafluoroethane (HFC-134a), pentane, n-butane, iso-butane, propane, trans-1,3,3,3-tetrafluoropropene (HF0-1234ze), and mixtures thereof, preferably dimethylether (DME), 1,1-difluoroethane (HFC-152a), and mixtures thereof. In a preferred embodiment, the propellant is selected from the group consisting of nitrogen, carbon dioxide, pentane, n-butane, iso-butane, propane, and combinations thereof. In embodiments, the formulation described herein comprises, based on the total weight of the composition, from 0.5 wt.-% to 60 wt.-%, from 1.0 wt.-% to 50 wt.-%, or from 2.0 wt.-% to 40 wt.-% of propellants.

The present disclosure also relates to a method of improving the action of an antiperspirant or a deodorant, wherein the method comprises adding to the antiperspirant or the deodorant at least one compound selected from glyceryl ethers, glyceryl esters, sorbitan esters, isosorbide esters, and N-methyl cyclic glucamides according to formula (I),

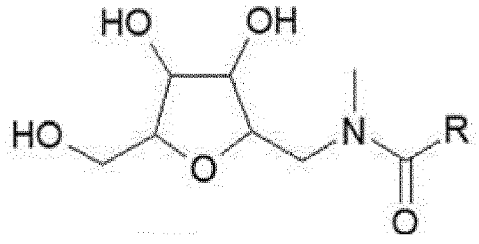

(I),

wherein R in Formula (I) is as defined elsewhere in this disclosure.

In embodiments of the method of improving the action of an antiperspirant or a deodorant disclosed herein, the antiperspirant or deodorant comprises at least one substance that is active against body odor. In embodiments, the at least one further substance that is active against body odor is selected from zinc ricinoleate, triethyl citrate, and mixtures thereof.

The present disclosure also relates to the use of at least one compound selected from glyceryl ethers, glyceryl esters, sorbitan esters, isosorbide esters, and N-methyl cyclic glucamides according to formula (I),

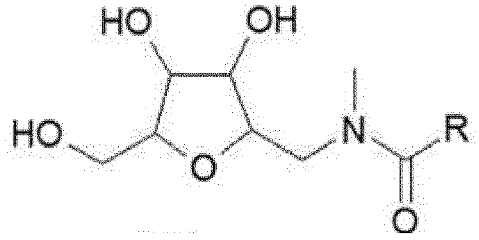

(I),

wherein R in Formula (I) is as defined elsewhere in this disclosure,
in an antiperspirant or a deodorant.

In embodiments, the antiperspirant or the deodorant comprises at least one further substance that is active against body odor. In embodiments, the at least one further substance that is active against body odor is selected from zinc ricinoleate, triethyl citrate, and mixtures thereof.

The present disclosure also relates to the use of at least one compound selected from glyceryl ethers, glyceryl esters, sorbitan esters, isosorbide esters, and N-methyl cyclic glucamides according to formula (I),

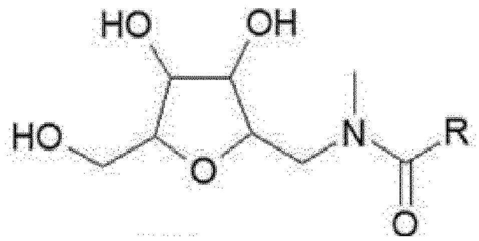

(I),

wherein R in Formula (I) is as defined elsewhere in this disclosure,
to improve the action of an antiperspirant or a deodorant.

In embodiments, the antiperspirant or the deodorant comprises at least one further substance that is active against body odor. In embodiments, the at least one further substance that is active against body odor is selected from zinc ricinoleate, triethyl citrate, and mixtures thereof.

The present disclosure also relates to the use of at least one compound selected from glyceryl ethers, glyceryl esters, sorbitan esters, isosorbide esters, and N-methyl cyclic glucamides according to formula (I)

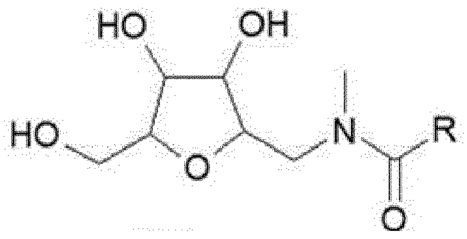

(I),

wherein R in Formula (I) is as defined elsewhere in this disclosure,
to inhibit the growth of certain odor-causing microbes.

Examples of odor-causing microbes include some species of bacteria or fungi, such as *Brevibacterium linens, Corynebacterium jeikeium,* and *Corynebacterium xerosis.*

The present disclosure also relates to the use of at least one compound selected from glyceryl ethers, glyceryl esters, sorbitan esters, isosorbide esters, and N-methyl cyclic glucamides according to formula (I)

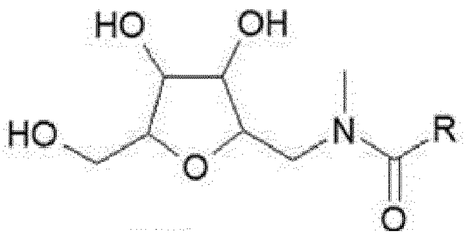

(I),

wherein R in Formula (I) is as defined elsewhere in this disclosure,

and at least one further substance that is active against body odor

to inhibit the growth of certain odor-causing microbes.

Examples of odor-causing microbes are as provided hereinabove. In embodiments, the at least one further substance that is active against body odor is selected from zinc ricinoleate, triethyl citrate, and mixtures thereof.

The present disclosure also relates to the use of at least one compound selected from glyceryl ethers, glyceryl esters, sorbitan esters, isosorbide esters, and N-methyl cyclic glucamides according to formula (I),

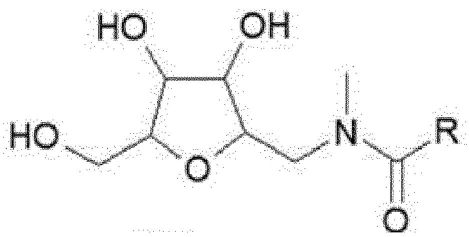

(I),

wherein R in Formula (I) is as defined elsewhere in this disclosure,
as an antimicrobial agent.

The present disclosure also relates to the use of at least one compound selected from glyceryl ethers, glyceryl esters, sorbitan esters, isosorbide esters, and N-methyl cyclic glucamides according to formula (I),

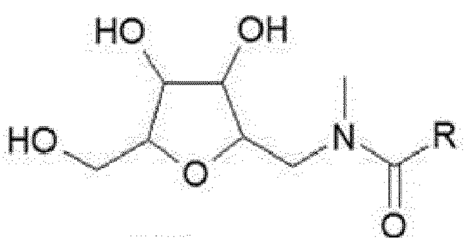

(I),

wherein R in Formula (I) is as defined elsewhere in this disclosure,

and at least one further substance that is active against body odor,

as an antimicrobial agent.

In embodiments, the at least one further substance that is active against body odor is selected from zinc ricinoleate, triethyl citrate, and mixtures thereof.

The present disclosure also relates to the use of at least one compound selected from glyceryl ethers, glyceryl esters, sorbitan esters, isosorbide esters, and N-methyl cyclic glucamides according to formula (I),

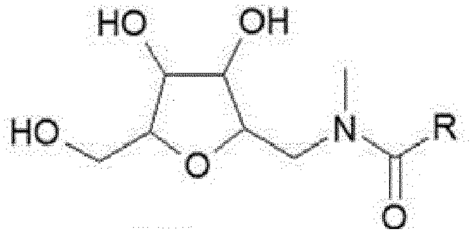

(I),

wherein R in Formula (I) is as defined elsewhere in this disclosure,
for preventing or reducing body odor.

The present disclosure also relates to the use of at least one compound selected from glyceryl ethers, glyceryl esters, sorbitan esters, isosorbide esters, and N-methyl cyclic glucamides according to formula (I),

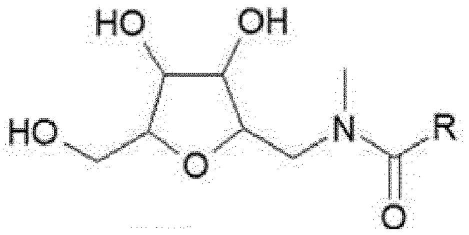

(I),

wherein R in Formula (I) is as defined elsewhere in this disclosure,

and at least one further substance that is active against body odor,

for preventing or reducing body odor.

In embodiments, the at least one further substance that is active against body odor is selected from zinc ricinoleate, triethyl citrate, and mixtures thereof.

The present disclosure also relates to at least one compound selected from glyceryl ethers, glyceryl esters, sorbitan esters, isosorbide esters, and N-methyl cyclic glucamides according to formula (I),

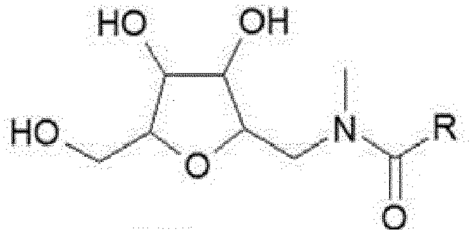

(I),

wherein R in Formula (I) is as defined elsewhere in this disclosure,
for the use of preventing or reducing body odor.

The present disclosure also relates to at least one compound selected from glyceryl ethers, glyceryl esters, sorbitan esters, isosorbide esters, and N-methyl cyclic glucamides according to formula (I),

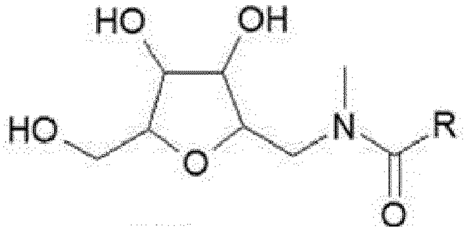

(I),

wherein R in Formula (I) is as defined elsewhere in this disclosure,

and at least one further substance that is active against body odor,

for the use of preventing or reducing body odor.

In embodiments, the at least one further substance that is active against body odor is selected from zinc ricinoleate, triethyl citrate, and mixtures thereof.

## EXAMPLES

Embodiments of the present disclosure will be further clarified by the following examples.

### Materials

Velsan® CGE is commercially available from Clariant. Chemical name: Caprylyl glyceryl ether.

Velsan® Flex is commercially available from Clariant. Chemical name: Capryloyl/Caproyl Anhydro Methyl Glucamide (and) Water, active content: 70 wt.-%. Capryloyl/Caproyl Anhydro Methyl Glucamide is a mixture of compounds of Formula (I) wherein R is $-(CH_2)_8CH_3$ and compounds of Formula (I) wherein R is $-(CH_2)_6CH_3$.

Velsan® EHG S is commercially available from Clariant. Chemical name: Ethylhexylglycerin.

Zinc ricinoleate and triethyl citrate are commercially available from Evonik and Jungbunzlauer, respectively.

### Assessment of Antibacterial Efficacy

Samples comprising (A) Velsan® CGE, Velsan® Flex, or Velsan® EHG S and/or (B) zinc ricinoleate or triethyl citrate were prepared as Examples 1-6. Herein, (A) and (B) may be referred to individually as a "deodorant active" or, collectively, as "deodorant actives."

The antibacterial efficacy of the samples was assessed by challenging each sample formulation with a prescribed inoculum of a suitable microorganism, storing the inoculated samples at a prescribed temperature, withdrawing samples from the container at specified intervals of time and counting the organisms in the samples so removed. The antimicrobial properties of a sample are adequate if, in the conditions of the test, there is a significant fall or no increase, as appropriate, in the number of microorganisms in the inoculated preparation after the times and at the temperatures prescribed.

As representative microorganisms the following test bacteria were used: *Brevibacterium linens* DSM 20425 (gram positive organism), *Corynebacterium jeikeium* DSM 7112 (gram positive organism), *Corynebacterium xerosis* DSM 20743 (gram positive organism), and *Staphylococcus hominis* DSM 20328 (gram positive organism).

The challenge tests were carried out according to the following procedure:

*Preparation of inoculum suspensions:*

*Brevibacterium linens, Corynebacterium jeikeium,* and *Corynebacterium xerosis.* The bacterial cultures were grown on oblique agar tubes consisting of tryptic soy ("TS")-Agar + 0.5% Tween 80 + 0.1% Lecithin. The cultures were incubated for 18-48 h, at 30 °C ± 2.5 °C. After 18-24 h, the cultures were checked for lush growth; if no growth, a 48h culture was used or the cultivation was repeated. 9 mL ± 0.1 mL of a 0.9% NaCl + 0.05% Tween solution was applied to the respective organism. The cells were carefully scraped off the agar surface with an inoculation loop and carefully removed from the oblique agar shortly after the vortexer. The bacteria suspension was decanted into an empty 50 mL centrifuge tube with a standing edge and mixed on the vortexer for about 1 minute. The inoculum was adjusted to a strength of 1-5 $\times$ $10^7$ CFU/mL.

*Staphylococcus hominis.* The bacterial culture was grown on oblique agar tubes consisting of TS agar. The culture was incubated for 18-24h h, at 30 °C ± 2.5 °C and checked for lush growth. 9 mL ± 0.1 mL of a 0.9% NaCl

solution was applied to the organism. The cells were carefully scraped off the agar surface with an inoculation loop and mixed with the vortexer to form a cell suspension. The inoculum was adjusted to a strength of 1-5 × $10^7$ CFU/mL.

*Preparation of samples:*

Each sample of Examples 1-6 was inoculated with 200 μL of the inoculum suspension of an organism and the time was recorded.

*Microbial count determinations of the samples:*

The bacterial count determinations of each sample were carried out after 3 hours, 6 hours, 24 hours, and/or 48 hours.

The number of ten-fold serial dilutions required for each sample was determined, with $10^{-1}$ to $10^{-4}$ dilutions being suitable for counting bacteria. Using a 1 mL flask stroke pipette and 9 mL dilution solution, the following procedure was used to create a dilution series:

1. Each time a dilution is made, it is mixed with the vortexer.
2. Transfer 1.0 ± 0.1 mL or 1.0 ± 0.1 g of the undiluted sample to 9 mL dilution solution to produce a 10-1 dilution.
3. Transfer 1.0 ± 0.1 mL of the $10^{-1}$ dilution to the petri dish labeled $10^{-1}$.
4. Transfer 1.0 ± 0.1 mL of the $10^{-1}$ dilution to 9 mL of dilution solution to produce a $10^{-2}$ dilution.
5. Transfer 1.0 ± 0.1 mL of the $10^{-2}$ dilution into the petri dish labeled $10^{-2}$.
6. Transfer 1.0 ± 0.1 mL of the $10^{-2}$ dilution to 9 mL of dilution solution to produce a $10^{-3}$ dilution.
7. Transfer 1.0 ± 0.1 mL of the $10^{-3}$ dilution to the petri dish labeled $10^{-3}$.
8. Transfer 1.0 ± 0.1 mL of the $10^{-3}$ dilution to 9 mL dilution solution to produce a $10^{-4}$ dilution.
9. Transfer 1.0 ± 0.1 mL of the $10^{-4}$ dilution to the petri dish labeled $10^{-4}$.

Once all dilutions were complete, the appropriate agar was added to the petri dishes. For samples inoculated with *Brevibacterium linens, Corynebacterium jeikeium,* or *Corynebacterium xerosis* TS-Agar +0.5% Tween was used. The dilution solution was the 0.9% NaCl +0.05% Tween solution. The samples inoculated with *Staphylococcus hominis* DSM 20328 were diluted with 0.9% NaCl solution and the plates were poured with TS-Agar. The petri dishes were incubated at 30.5°C ± 2.5°C for 4-5 days.

The challenge test results are presented as the logarithmic reduction in bacterial count as measured at various intervals of time.

Test System 1 was used to prepare Example 1 (Samples 1-9) and Example 2 (Samples 10-18).

**Test System 1**

| Phase | Ingredients | Weight Percent |
|---|---|---|
| **A** | Plantasens Olive LD (Clariant) (*Hydrogenated Ethylhexyl Olivate (and) Hydrogenated Olive Oil Unsaponifiables*) | 2.00 % |
| | Cetiol OE *(Dicaprylyl Ether*) | 4.00 % |
| | Eutanol G (*Octyldodecanol*) | 2.00 % |
| | Genapol HS 200 (Clariant) (*Steareth-20*) | 3.00 % |
| | Genapol HS 20 (*Steareth-2*) | 1.20 % |
| **B** | Zinc ricinoleate | Q.s. |
| | Triethyl citrate | Q.s. |
| | **Velsan CGE** (*Caprylyl Glyceryl Ether*) | Q.s. |
| **C** | Xanthan Gum FNCSP-PC (*Xantham Gum*) | 0.05 % |
| | Glycerin | 2.00 % |
| | Water | ad 100 % |

**Example 1, pH = 5.5**

| | | | Logarithmic Reduction in Bacterial Count | | | | | | | | | | | |
|---|---|---|---|---|---|---|---|---|---|---|---|---|---|---|
| | Deodorant Actives (Wt. %) | | *Brevibacterium linens* | | | *Corynebacterium jeikeium* | | | *Corynebacterium xerosis* | | | *Staphylococcus hominis* | | |
| Sample | (A) | (B) | 6h | 24h | 48h | 6h | 24h | 48h | 6h | 24h | 48h | 6h | 24h | 48h |
| 1 | - | - | 0.87 | 2.01 | 3.22 | -0.13 | 0.12 | 0.35 | 1.19 | 2.33 | 2.84 | -0.09 | 0.03 | 0.31 |
| 2 | - | Zinc ricinoleate (3.0) | 2.24 | 4.79 | 4.79 | 1.38 | 4.89 | 4.89 | 2.33 | 5.72 | 5.72 | 0.11 | 3.61 | 3.66 |
| 3 | - | Zinc ricinoleate (1.5) | 1.98 | 4.79 | 4.79 | 1.15 | 4.89 | 4.89 | 2.22 | 5.72 | 5.72 | 0.17 | 2.38 | 3.66 |
| 4 | - | Zinc ricinoleate (1.0) | 1.88 | 4.79 | 4.79 | 1.10 | 4.89 | 4.89 | 2.02 | 5.72 | 5.72 | -1.00 | 1.00 | 3.31 |
| 5 | - | Zinc ricinoleate (0.5) | 1.13 | 2.59 | 3.16 | 0.17 | 2.15 | 4.89 | 1.87 | 4.97 | 5.72 | -0.05 | 0.83 | 3.14 |
| 6 | Velsan CGE (1.0) | Zinc ricinoleate (1.5) | 4.79 | 4.79 | 4.79 | 4.89 | 4.89 | 4.89 | 5.19 | 5.72 | 5.72 | 2.23 | 3.66 | 3.66 |
| 7 | Velsan CGE (1.0) | Zinc ricinoleate (1.0) | 4.79 | 4.79 | 4.79 | 4.89 | 4.89 | 4.89 | 5.72 | 5.72 | 5.72 | 2.04 | 3.66 | 3.66 |
| 8 | Velsan CGE (1.0) | Zinc ricinoleate (0.5) | 4.26 | 4.79 | 4.79 | 4.89 | 4.89 | 4.89 | 5.72 | 5.72 | 5.72 | 2.66 | 3.66 | 3.66 |
| 9 | Velsan CGE (1.0) | - | 3.63 | 4.79 | 4.79 | 4.00 | 4.89 | 4.89 | 4.56 | 5.72 | 5.72 | 0.11 | 0.56 | 1.15 |

**Example 2, pH = 5.5**

| | | | Logarithmic Reduction in Bacterial Count | | | | | | | | | | | | |
|---|---|---|---|---|---|---|---|---|---|---|---|---|---|---|---|
| | Deodorant Actives (Wt. %) | | *Brevibacterium linens* | | | | *Corynebacterium jeikeium* | | | | *Staphylococcus hominis* | | | |
| Sample | (A) | (B) | 3h | 6h | 24h | 48h | 3h | 6h | 24h | 48h | 3h | 6h | 24h | 48h |
| 10 | - | - | 1.14 | 1.68 | 3.36 | 3.41 | 1.47 | 0.88 | 1.61 | 1.94 | 1.47 | 0.88 | 1.61 | 1.94 |
| 11 | - | Triethyl citrate (5.0) | 3.36 | 3.41 | 3.41 | 3.41 | 4.38 | 4.38 | 4.73 | 4.73 | 4.38 | 4.38 | 4.73 | 4.73 |
| 12 | - | Triethyl citrate (3.0) | 2.11 | 2.88 | 3.41 | 3.41 | 3.00 | 3.73 | 4.73 | 4.73 | 3.00 | 3.73 | 4.73 | 4.73 |
| 13 | - | Triethyl citrate (2.0) | 1.61 | 2.25 | 3.41 | 3.41 | 2.66 | 3.43 | 4.73 | 4.73 | 2.66 | 3.43 | 4.73 | 4.73 |
| 14 | - | Triethyl citrate (1.0) | 0.96 | 1.82 | 3.41 | 3.41 | 1.14 | 2.15 | 3.43 | 4.73 | 1.14 | 2.15 | 3.43 | 4.73 |
| 15 | Velsan CGE (1.0) | Triethyl citrate (3.0) | 3.36 | 3.41 | 3.41 | 3.41 | 4.38 | 4.68 | 4.73 | 4.73 | 4.38 | 4.68 | 4.73 | 4.73 |
| 16 | Velsan CGE (1.0) | Triethyl citrate (2.0) | 3.06 | 3.41 | 3.41 | 3.41 | 4.20 | 4.38 | 4.73 | 4.73 | 4.20 | 4.38 | 4.73 | 4.73 |
| 17 | Velsan CGE (1.0) | Triethyl citrate (1.0) | 2.76 | 3.41 | 3.41 | 3.41 | 4.30 | 4.73 | 4.73 | 4.73 | 4.30 | 4.73 | 4.73 | 4.73 |
| 18 | Velsan CGE (1.0) | - | 1.69 | 3.06 | 3.41 | 3.41 | 1.88 | 3.10 | 4.73 | 4.73 | 1.88 | 3.10 | 4.73 | 4.73 |

Test System 2 was used to prepare Example 3 (Samples 19-27), Example 4 (Samples 28-36), and Example 5 (Samples 37-45).

**Test System 2**

| Phase | Ingredients | Weight Percent |
|---|---|---|
| A | Cetiol MM (*Myrisyl Myristate*) | 1.00 % |
| | Lanette 0 (*Cetearyl Alcohol*) | 2.00 % |
| | Isopropyl Palmitate | 1.00 % |

(continued)

| Phase | Ingredients | Weight Percent |
|---|---|---|
| | Imwitor 372 P (*Glyceryl Stearate Citrate*) | 3.00 % |
| B | Zinc ricinoleate | Q.s. |
| | Triethyl citrate | Q.s. |
| | **Velsan CGE** (*Caprylyl Glyceryl Ether*)<br>**or**<br>**Velsan EHG S** (*Ethylhexylglycerin*) | Q.s. |
| C | Xanthan Gum FNCSP-PC (*Xantham Gum*) | 0.50 % |
| | Glycerin | 3.00 % |
| | D-Panthenol | 1.00 % |
| | Water | ad 100 % |

**Example 3, pH = 5.5**

| | Deodorant Actives (Wt. %) | | Logarithmic Reduction in Bacterial Count | | | | | | | | | | | |
|---|---|---|---|---|---|---|---|---|---|---|---|---|---|---|
| | | | *Brevibacterium linens* | | | | *Corynebacterium jeikeium* | | | | *Staphylococcus hominis* | | | |
| **Sample** | **(A)** | **(B)** | **3h** | **6h** | **24h** | **48h** | **3h** | **6h** | **24h** | **48h** | **3h** | **6h** | **24h** | **48h** |
| **19** | - | - | 0.18 | 0.11 | 0.30 | 0.23 | -0.09 | -0.33 | 0.10 | -0.01 | 0.02 | 0.02 | 0.12 | 0.12 |
| **20** | - | Zinc ricinoleate (3.0) | 0.28 | 0.18 | 1.46 | 1.88 | 0.86 | 1.66 | 3.92 | 4.56 | 0.18 | 0.46 | 1.50 | 3.30 |
| **21** | - | Zinc ricinoleate (1.5) | 0.02 | 0.21 | 1.52 | 1.99 | 0.94 | 1.48 | 4.52 | 4.56 | 0.32 | 0.20 | 1.26 | 3.15 |
| **22** | - | Zinc ricinoleate (1.0) | 0.30 | 0.44 | 1.81 | 2.21 | 0.90 | 1.68 | 4.52 | 4.56 | 0.20 | 0.43 | 1.14 | 2.92 |
| **23** | - | Zinc ricinoleate (0.5) | 0.01 | 0.12 | 1.58 | 2.23 | 0.55 | 0.93 | 3.37 | 4.56 | 0.17 | 0.22 | 0.46 | 1.16 |
| **24** | Velsan CGE (1.0) | Zinc ricinoleate (1.5) | 3.06 | 4.08 | 4.60 | 4.60 | 4.56 | 4.56 | 4.56 | 4.56 | 1.90 | 4.34 | 4.34 | 4.34 |
| **25** | Velsan CGE (1.0) | Zinc ricinoleate (1.0) | 2.82 | 4.56 | 4.60 | 4.60 | 4.56 | 4.56 | 4.56 | 4.56 | 1.82 | 4.00 | 4.34 | 4.34 |
| **26** | Velsan CGE (1.0) | Zinc ricinoleate (0.5) | 3.18 | 4.60 | 4.60 | 4.60 | 4.56 | 4.56 | 4.56 | 4.56 | 1.19 | 1.92 | 4.00 | 4.34 |
| **27** | Velsan CGE (1.0) | - | 2.45 | 3.04 | 4.56 | 4.60 | 4.52 | 4.56 | 4.56 | 4.56 | 0.13 | 0.18 | 0.34 | 0.83 |

**Example 4, pH = 5.5**

| | Deodorant Actives (Wt. %) | | Logarithmic Reduction in Bacterial Count | | | | | | | | | | | |
|---|---|---|---|---|---|---|---|---|---|---|---|---|---|---|
| | | | *Brevibacterium linens* | | | | *Corynebacterium jeikeium* | | | | *Staphylococcus hominis* | | | |
| **Sample** | **(A)** | **(B)** | **3h** | **6h** | **24h** | **48h** | **3h** | **6h** | **24h** | **48h** | **3h** | **6h** | **24h** | **48h** |
| **28** | - | - | 0.17 | 0.23 | 0.45 | 0.42 | 0.02 | 0.01 | 0.20 | 0.20 | 0.16 | -0.02 | 0.09 | 0.03 |
| **29** | - | Triethyl citrate (5.0) | 3.08 | 4.77 | 4.77 | 4.77 | 4.89 | 4.89 | 4.89 | 4.89 | 0.21 | 0.31 | 0.57 | 0.87 |
| **30** | - | Triethyl citrate (3.0) | 1.83 | 4.25 | 4.77 | 4.77 | 2.54 | 3.77 | 4.89 | 4.89 | 0.14 | 0.12 | 0.22 | 0.44 |
| **31** | - | Triethyl citrate (2.0) | 0.92 | 2.84 | 4.72 | 4.77 | 1.06 | 2.00 | 4.89 | 4.89 | 0.20 | 0.16 | 0.02 | 0.21 |
| **32** | - | Triethyl citrate (1.0) | 0.53 | 1.69 | 4.25 | 4.42 | 0.35 | 0.45 | 1.80 | 2.38 | 0.12 | 0.07 | 0.13 | 0.00 |
| **33** | Velsan CGE (1.0) | Triethyl citrate (3.0) | 4.77 | 4.77 | 4.77 | 4.77 | 4.89 | 4.89 | 4.89 | 4.89 | 3.04 | 3.67 | 5.01 | 5.01 |
| **34** | Velsan CGE (1.0) | Triethyl citrate (2.0) | 4.77 | 4.77 | 4.77 | 4.77 | 4.89 | 4.89 | 4.89 | 4.89 | 2.09 | 2.81 | 4.17 | 4.71 |
| **35** | Velsan CGE (1.0) | Triethyl citrate (1.0) | 4.77 | 4.77 | 4.77 | 4.77 | 4.89 | 4.89 | 4.89 | 4.89 | 0.91 | 1.47 | 2.97 | 3.81 |
| **36** | Velsan CGE (1.0) | - | 4.77 | 4.77 | 4.77 | 4.77 | 4.89 | 4.89 | 4.89 | 4.89 | 0.75 | 1.43 | 2.67 | 3.18 |

**Example 5, pH = 5.5**

| | Deodorant Actives (Wt. %) | | Logarithmic Reduction in Bacterial Count | | | | | | | | | | | |
|---|---|---|---|---|---|---|---|---|---|---|---|---|---|---|
| | | | *Brevibacterium linens* | | | | *Corynebacterium jeikeium* | | | | *Staphylococcus hominis* | | | |
| **Sample** | **(A)** | **(B)** | **3h** | **6h** | **24h** | **48h** | **3h** | **6h** | **24h** | **48h** | **3h** | **6h** | **24h** | **48h** |
| **37** | - | - | 0.18 | 0.11 | 0.30 | 0.23 | -0.09 | -0.33 | 0.10 | -0.01 | 0.02 | 0.02 | 0.12 | 0.12 |
| **38** | - | Zinc ricinoleate (3.0) | 0.28 | 0.18 | 1.46 | 1.88 | 0.86 | 1.66 | 3.92 | 4.56 | 0.18 | 0.46 | 1.50 | 3.30 |
| **39** | - | Zinc ricinoleate (1.5) | 0.02 | 0.21 | 1.52 | 1.99 | 0.94 | 1.48 | 4.52 | 4.56 | 0.32 | 0.20 | 1.26 | 3.15 |
| **40** | - | Zinc ricinoleate (1.0) | 0.30 | 0.44 | 1.81 | 2.21 | 0.90 | 1.68 | 4.52 | 4.56 | 0.20 | 0.43 | 1.14 | 2.92 |
| **41** | - | Zinc ricinoleate (0.5) | 0.01 | 0.12 | 1.58 | 2.23 | 0.55 | 0.93 | 3.37 | 4.56 | 0.17 | 0.22 | 0.46 | 1.16 |
| **42** | Velsan EHG S (1.0) | Zinc ricinoleate (1.5) | 3.21 | 4.60 | 4.60 | 4.60 | 4.56 | 4.56 | 4.56 | 4.56 | 2.88 | 4.34 | 4.34 | 4.34 |
| **43** | Velsan EHG S (1.0) | Zinc ricinoleate (1.0) | 3.04 | 4.60 | 4.60 | 4.60 | 4.56 | 4.56 | 4.56 | 4.56 | 2.58 | 4.34 | 4.34 | 4.34 |
| **44** | Velsan EHG S (1.0) | Zinc ricinoleate (0.5) | 3.33 | 4.60 | 4.60 | 4.60 | 4.56 | 4.56 | 4.56 | 4.56 | 2.10 | 4.34 | 4.34 | 4.34 |
| **45** | Velsan EHG S (1.0) | - | 2.45 | 3.04 | 4.56 | 4.60 | 4.52 | 4.56 | 4.56 | 4.56 | 0.13 | 0.18 | 0.34 | 0.83 |

Test System 3 was used to prepare Example 6 (Samples 46-52).

| Phase | Ingredients | Weight Percent |
|---|---|---|
| A | Cetiol MM (*Myrisyl Myristate*) | 1.00 % |
| | Lanette 0 (*Cetearyl Alcohol*) | 2.00 % |
| | Isopropyl Palmitate | 1.00 % |
| | Imwitor 372 P (*Glyceryl Stearate Citrate*) | 3.00 % |
| B | **Velsan Flex** | Q.s. |
| C | Xanthan Gum FNCSP-PC (*Xantham Gum*) | 0.50 % |
| | Glycerin | 3.00 % |
| | D-Panthenol | 1.00 % |
| | Water | ad 100 % |

**Example 6, pH = 5.5**

| | | Logarithmic Reduction in Bacterial Count | | | | | | | | | | | |
|---|---|---|---|---|---|---|---|---|---|---|---|---|---|
| | Deodorant Active (Wt. %) | *Brevibacterium linens* | | | | *Corynebacterium jeikeium* | | | | *Staphylococcus hominis* | | | |
| Sample | (A) only | 3h | 6h | 24h | 48h | 3h | 6h | 24h | 48h | 3h | 6h | 24h | 48h |
| 46 | - | 0.24 | 0.65 | 1.64 | 2.28 | 0.04 | -0.01 | -0.01 | 0.16 | -0.14 | -0.02 | -0.01 | -0.02 |
| 47 | Velsan Flex (0.20) | 0.76 | 2.00 | 4.59 | 4.59 | 0.50 | 1.55 | 4.13 | 4.78 | -0.06 | 0.03 | 0.08 | 0.12 |
| 48 | Velsan Flex (0.40) | 3.70 | 4.59 | 4.59 | 4.59 | 4.43 | 4.78 | 4.78 | 4.78 | 0.22 | 0.28 | 0.48 | 0.61 |
| 49 | Velsan Flex (0.60) | 4.24 | 4.59 | 4.59 | 4.59 | 4.78 | 4.78 | 4.78 | 4.78 | 0.53 | 0.54 | 0.84 | 0.93 |
| 50 | Velsan Flex (0.80) | 4.59 | 4.59 | 4.59 | 4.59 | 4.78 | 4.78 | 4.78 | 4.78 | 0.49 | 0.65 | 0.94 | 1.12 |
| 51 | Velsan Flex (1.00) | 4.59 | 4.59 | 4.59 | 4.59 | 4.78 | 4.78 | 4.78 | 4.78 | 0.63 | 0.69 | 0.95 | 1.36 |
| 52 | Velsan Flex (1.20) | 4.59 | 4.59 | 4.59 | 4.59 | 4.78 | 4.78 | 4.78 | 4.78 | 0.61 | 0.75 | 0.99 | 1.39 |

The samples containing at least one deodorant active (A) or (B) (Samples 2-8, 11-18, 20-27, 29-36, 38-45, and 47-52) consistently resulted in a larger logarithmic decrease in bacterial count, particularly for the first six hours, than the samples that ***did not*** contain a deodorant active (Samples 1, 10, 19, 28, 37, and 46).

The data further reveal that ***the antibacterial properties of the deodorant actives (A) are generally superior to those of the deodorant actives (B),*** especially for the first six hours. For instance, the samples containing only 1.0 wt.-% of Velsan CGE or Velsan EHG S (Samples 9, 18, 27, 36, and 45) were all more effective at inhibiting bacterial growth than the samples containing only 1.0 wt.-% of zinc ricinoleate or triethyl citrate (Samples 4, 14, 22, 32, and 40), up to at least the 6-hour mark. The samples containing only Velsan Flex (Example 6, Samples 47-52) exhibited dramatically reduced bacterial growth, especially for the first six hours, even at relatively low doses (*e.g.,* Samples 48 and 49).

Most importantly, however, the data indicate that ***certain compositions comprising a combination of deodorant actives (A) and (B) display an enhanced antibacterial effect*** relative to compositions containing only deodorant active (A) or deodorant active (B). Specifically, Samples 6-8, 15-17, 24-26, 33-35, and 42-44, which contained zinc ricinoleate or triethyl citrate (a deodorant active (B)) **plus** Velsan CGE or Velsan EHG S (a deodorant active (A)), showed improved bacterial growth inhibition relative to the samples containing only a deodorant active (B) (Samples 2-5, 11-14, 20-23, 29-32, and 38-41) **or** a deodorant active (A) (Samples 9, 18, 27, 36, and 45).

The above-described effect is most significant for at least the first six hours, but in many cases is observed even longer. As shown by the data, the "boost" in activity provided by the combination of the two deodorant actives means that ***the same antibacterial effect can be achieved at an overall lower dose of active ingredients.***

Finally, ***the deodorant actives and combinations thereof appear to show some selectivity for certain bacterial species.*** In particular, the inhibition of *Staphylococcus hominis* is much less remarkable compared to the other bacterial species tested.

It will be apparent to those skilled in the art that various modifications and variations can be made to the embodiments described herein without departing from the spirit and scope of the claimed subject matter. Thus, it is intended that the specification cover the modifications and variations of the various embodiments described herein provided such modification and variations come within the scope of the appended claims and their equivalents.

**Claims**

**1.** An antimicrobial composition comprising

(A) at least one compound selected from glyceryl ethers, glyceryl esters, sorbitan esters, isosorbide esters, and N-methyl cyclic glucamides according to formula (I),

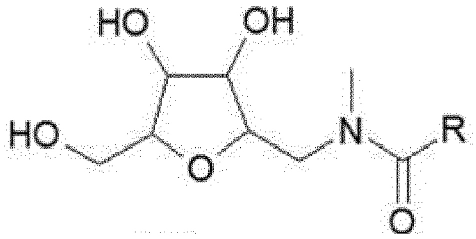

(I),

wherein R is a saturated hydrocarbon chain having 5 to 23 carbon atoms or an unsaturated hydrocarbon chain having 5 to 23 carbon atoms; and
(B) at least one further substance that is active against body odor.

2. The composition according to claim 1, wherein the composition comprises (A) at least one glyceryl ether selected from

a monoether of glycerin and a $C_6$-$C_{20}$ fatty alcohol; and
a diether of glycerin and one or more $C_6$-$C_{20}$ fatty alcohols.

3. The composition according to claim 1 or 2, wherein the composition comprises (A) at least one glyceryl ether selected from caprylyl glyceryl ether, ethylhexylglycerin, and methylheptylglycerin.

4. The composition according to any one of claims 1 to 3, wherein (B) is selected from zinc ricinoleate, triethyl citrate, and mixtures thereof.

5. The composition according to any one of claims 1 to 4, wherein the composition comprises (A) at least one glyceryl ester selected from glyceryl caprylate and glyceryl caprate.

6. The composition according to any one of claims 1 to 5, wherein the composition comprises (A) at least one sorbitan ester selected from

a monoester of sorbitan and a $C_6$-$C_{20}$ fatty acid;
a diester of sorbitan and one or more $C_6$-$C_{20}$ fatty acids; and
a triester of sorbitan and one or more $C_6$-$C_{20}$ fatty acids.

7. The composition according to any one of claims 1 to 6, wherein the composition comprises (A) at least one sorbitan ester selected from sorbitan caprylate, sorbitan stearate, sorbitan olivate, sorbitan oleate, sorbitan caprate, sorbitan laurate, sorbitan myristate, and sorbitan caproate.

8. The composition according to any one of claims 1 to 7, wherein the composition comprises (A) at least one isosorbide ester selected from

a monoester of isosorbide and a $C_6$-$C_{20}$ fatty acid; and
a diester of isosorbide and one or more $C_6$-$C_{20}$ fatty acids.

9. The composition according to any one of claims 1 to 8, wherein the composition comprises (A) at least one N-methyl cyclic glucamide according to Formula (I) wherein R in Formula (I) is a saturated hydrocarbon chain having 5 to 17 carbon atoms or an unsaturated hydrocarbon chain having 5 to 17 carbon atoms.

10. The composition according to any one of claims 1 to 9, wherein the composition is a blend comprising from 20 wt.-% to 70 wt.-% of (A) and from 30 wt.-% to 80 wt.-% of (B), based on the total weight of (A) and (B) in the blend.

11. The composition according to any one of claims 1 to 10, wherein the composition is a blend comprising (A) and (B) in a weight-to-weight ratio of from 10:1 to 1:10.

12. A method of improving the action of an antiperspirant or a deodorant, wherein the method comprises adding to the antiperspirant or the deodorant at least one compound selected from glyceryl ethers, glyceryl esters, sorbitan esters, isosorbide esters, and N-methyl cyclic glucamides according to formula (I),

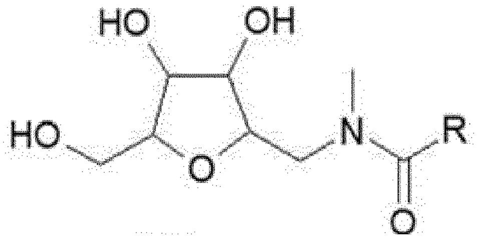

(I),

wherein R is a saturated hydrocarbon chain having 5 to 23 carbon atoms or an unsaturated hydrocarbon chain having 5 to 23 carbon atoms.

**13.** A formulation comprising

(A) from 0.1 wt.-% to 10 wt.-%, based on the total weight of the formulation, of at least one compound selected from glyceryl ethers, glyceryl esters, sorbitan esters, isosorbide esters, and N-methyl cyclic glucamides according to formula (I),

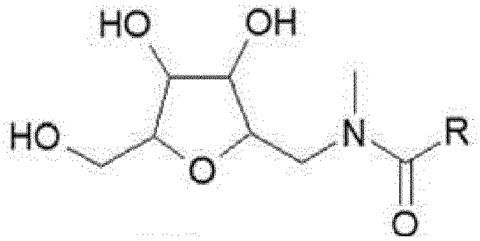

(I),

wherein R is a saturated hydrocarbon chain having 5 to 23 carbon atoms or an unsaturated hydrocarbon chain having 5 to 23 carbon atoms; and
(B) from 0.1 wt.-% to 10 wt.-%, based on the total weight of the formulation, of at least one further substance that is active against body odor.

**14.** The formulation according to claim 13, wherein (A) is caprylyl glyceryl ether and (B) is selected from zinc ricinoleate, triethyl citrate, and mixtures thereof.

**15.** Use of at least one compound selected from glyceryl ethers, glyceryl esters, sorbitan esters, isosorbide esters, and N-methyl cyclic glucamides according to formula (I),

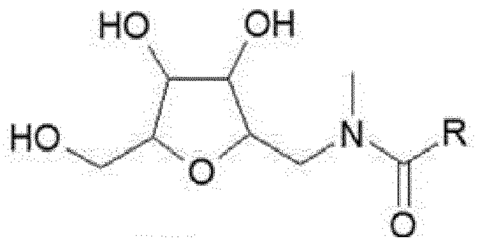

(I),

wherein R is a saturated hydrocarbon chain having 5 to 23 carbon atoms or an unsaturated hydrocarbon chain having 5 to 23 carbon atoms,
in an antiperspirant or a deodorant.